# FASCICULE DE BREVET EUROPEEN

(11) **EP 0 655 248 B1**
(45) Date de publication et mention de la délivrance du brevet: **15.09.1999**
(21) Numéro de dépôt: 94402673.1
(22) Date de dépôt: 23.11.1994
(51) Int. Cl.: A61K 31/495, A61K 31/505

(54) **Utilisation des dérivés de 1- 4-[4-aryl(ou hétéroaryl)-1-pipérazinyl]-butyl 1-H-azole pour la préparation de médicaments destinés au traitement des troubles de la sécrétion gastrique**
Verwendung der 1-4-4-Aryl(oder Heteroaryl)-1-Piperazinylbutyl-1H-Azol-Derivate zur Herstellung von Arzneimitteln zur Behandlung der Störungen der Magensekretion
Use of 1-4-4-aryl(or heteroaryl)-1-piperazinylbutyl-1H-azole derivatives for the preparation of medicaments for the treatment of disorders of gastric secretion

(30) Priorité: 25.11.1993 FR 9314102
(43) Date de publication de la demande: 31.05.1995
(73) Titulaire: LABORATORIOS DEL DR. ESTEVE, S.A., 08026 Barcelona (ES)
(72) Inventeur: Frigola Constansa, Jordi, E-08013 Barcelone (ES); Merce Vidal, Ramon, E-08014 Barcelone (ES)
(74) Mandataire: Ahner, Francis

(56) Documents cités:
- EP-A- 0 455 510
- THE JOURNAL OF PHARMACOLOGY AND EXPERIMENTAL THERAPEUTICS, vol.262, no.1, Juillet 1992 pages 90 - 98 COSTALL, B. ET AL 'PROFILE OF ACTION OF A NOVEL 5-HYDROXYTRYPTAMINE 1A RECEPTOR LIGAND E-4424 TO INHIBIT AVERSIVE BEHAVIOUR IN THE MOUSE, RAT AND MARMOSET'

## Description

La présente invention concerne l'utilisation des dérivés de 1-{4-[4-aryl(ou hétéroaryl)-1-pipérazinyl]-butyl}-1-*H*-azole ainsi que de leurs sels physiologiquement acceptables, pour la fabrication de médicaments destinés au traitement des troubles associés à la sécrétion gastrique.

Les composés auxquels se rapporte la présente invention ont été décrits dans les brevets européens EP 382637 et EP 497659 ainsi que dans le brevet européen EP 502786 qui concerne un procédé de préparation de dérivés d'aryl (ou hétéroaryl)-pipérazinyl-butyl-azoles. Dans les brevets EP 382637 et EP 497659, nous avons revendiqué l'utilisation de ces composés pour le traitement de certaines maladies du système nerveux central. Nous avons maintenant découvert que les dérivés d'aryl (ou hétéroaryl)-pipérazinyl-butyl-azoles montrent une activité antisécrétoire gastrique et que par conséquent ils sont utiles pour le traitement de l'hypersécrétion gastrique ou comme agents antiulcéreux. En particulier les composés sont appropriés pour prévenir ou traiter les maladies gastrointestinales des mammifères, l'homme inclus.

Les composés préconisés dans le cadre de la présente invention répondent à la formule générale I 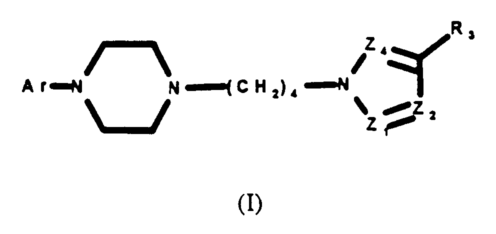 dans laquelle:
Ar représente un radical aromatique azoté ou non, choisi parmi les aryles différemment substitués, la 2-pyrimidine différemment substituée, et le 3-(1,2-benzisothiazole).
Z₁ représente un atome d'azote ou un atome de carbone substitué ou non qu'on peut représenter par: C-R₁.
Z₂ représente un atome d'azote ou un atome de carbone substitué ou non qu'on peut représenter par: C-R₂.
Z₄ représente un atome d'azote ou un atome de carbone substitué ou non qu'on peut représenter par: C-R₄.
et R₁, R₂, R₃ et R₄, identiques ou différents, pouvant également former une partie d'un autre cycle, aromatique ou non, représentent un atome d'hydrogène, un halogène, un radical alkyle inférieur, un radical nitro, un radical hydroxy, un radical alcoxy, un radical cyano, un radical carboxylique, un radical carboxamido, un radical carboxylate d'alkyle, un radical aryle ou aryle substitué, un radical sulfonique, un radical sulfonamido, substitué ou non sur le groupement amino, un radical amino ou amino substitué.

Les composés identifiés par les exemples 1 à 84 sont obtenus par les procédures décrites dans les brevets EP 382637, EP 497659 et EP 502786, et les données pour leur identification sont exposées dans le tableau I. Les exemples suivants illustrent les propriétés de quelques dérivés entrant dans le cadre de la présente invention.

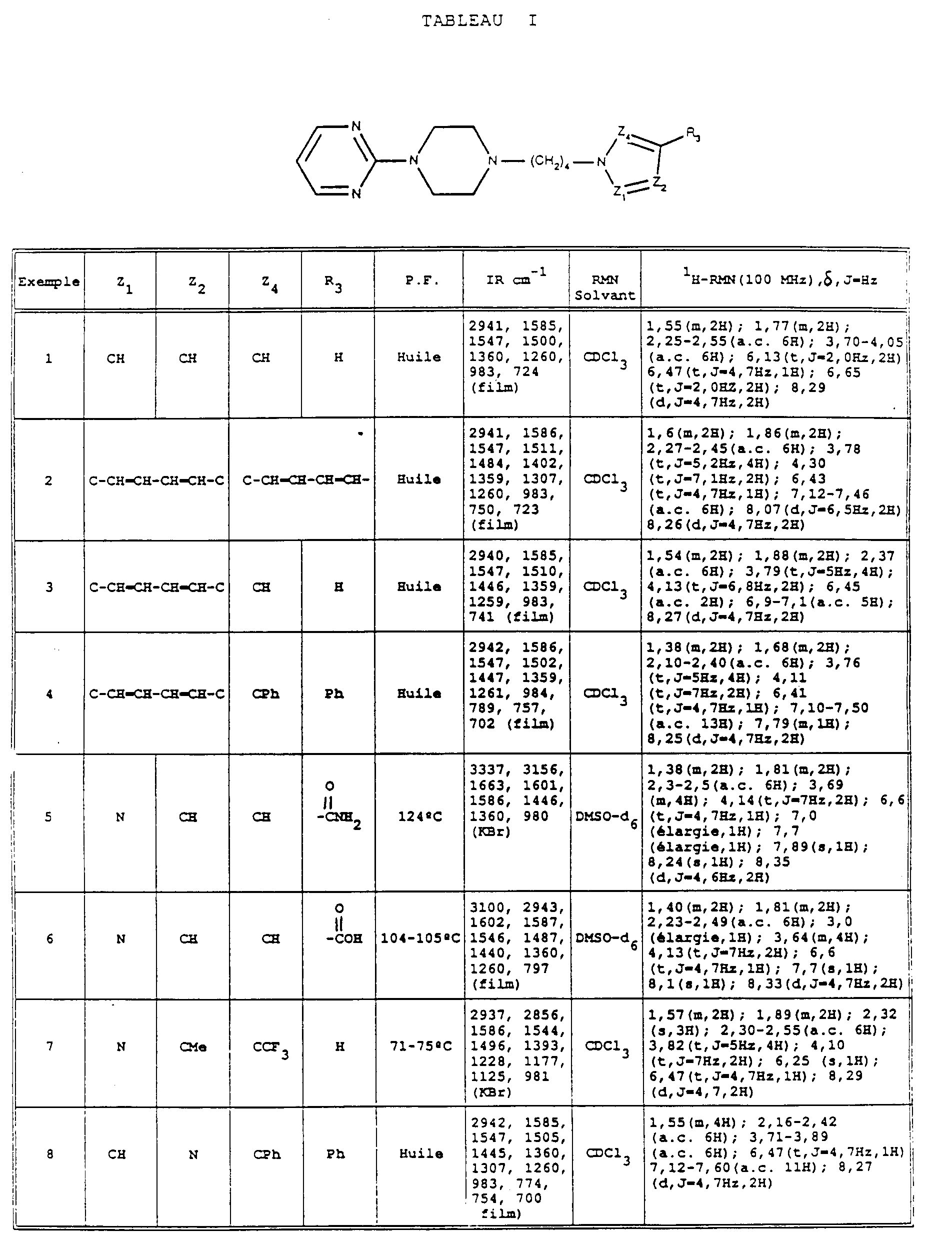

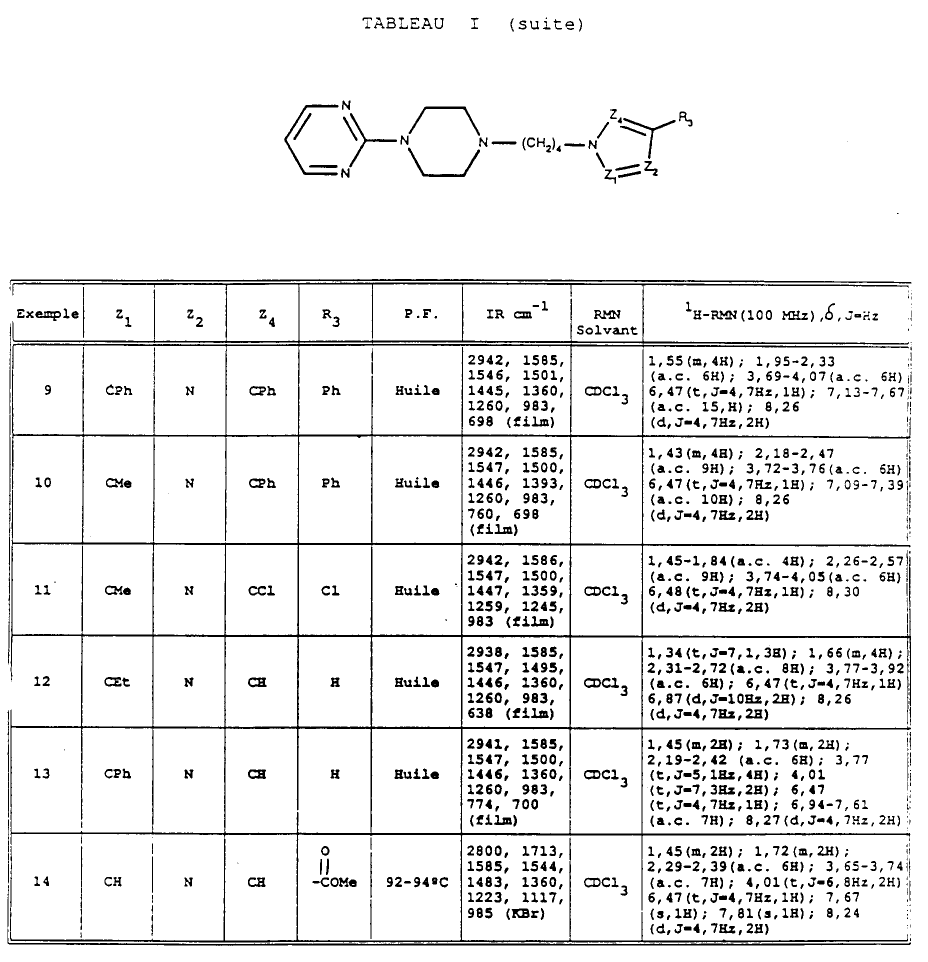

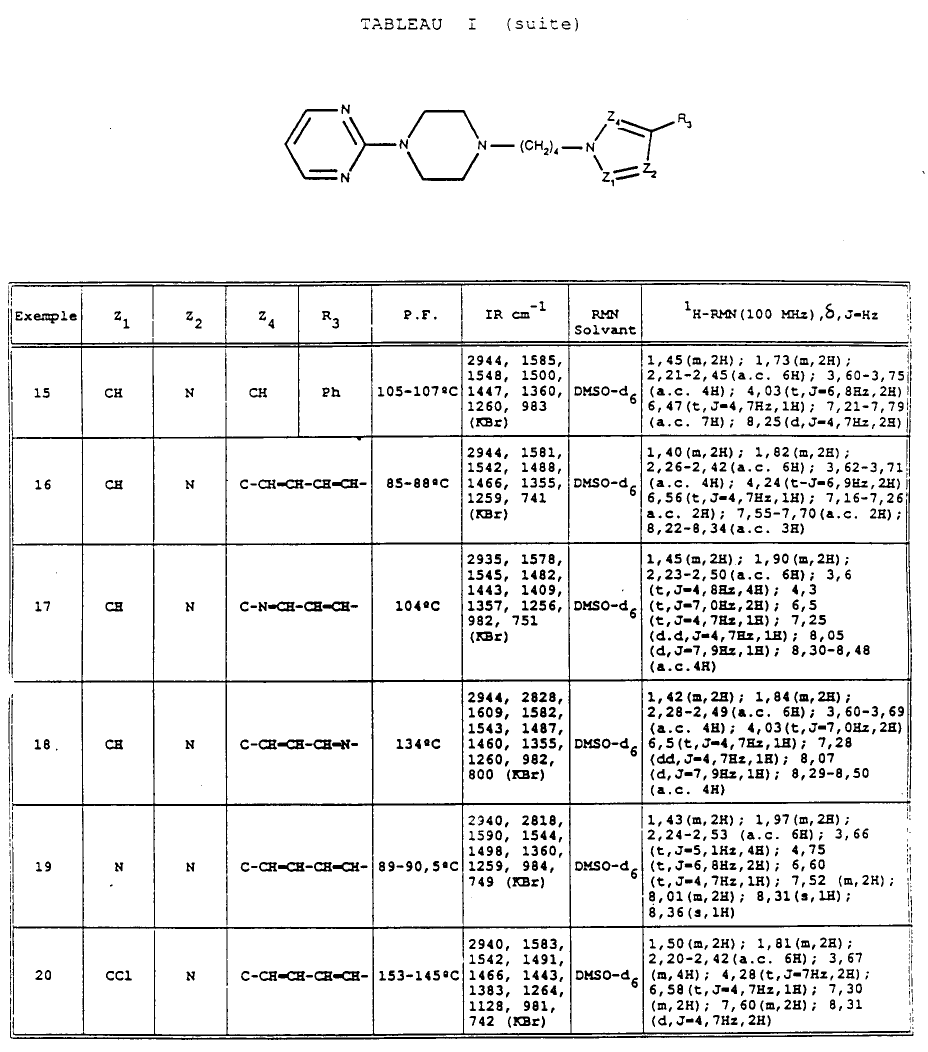

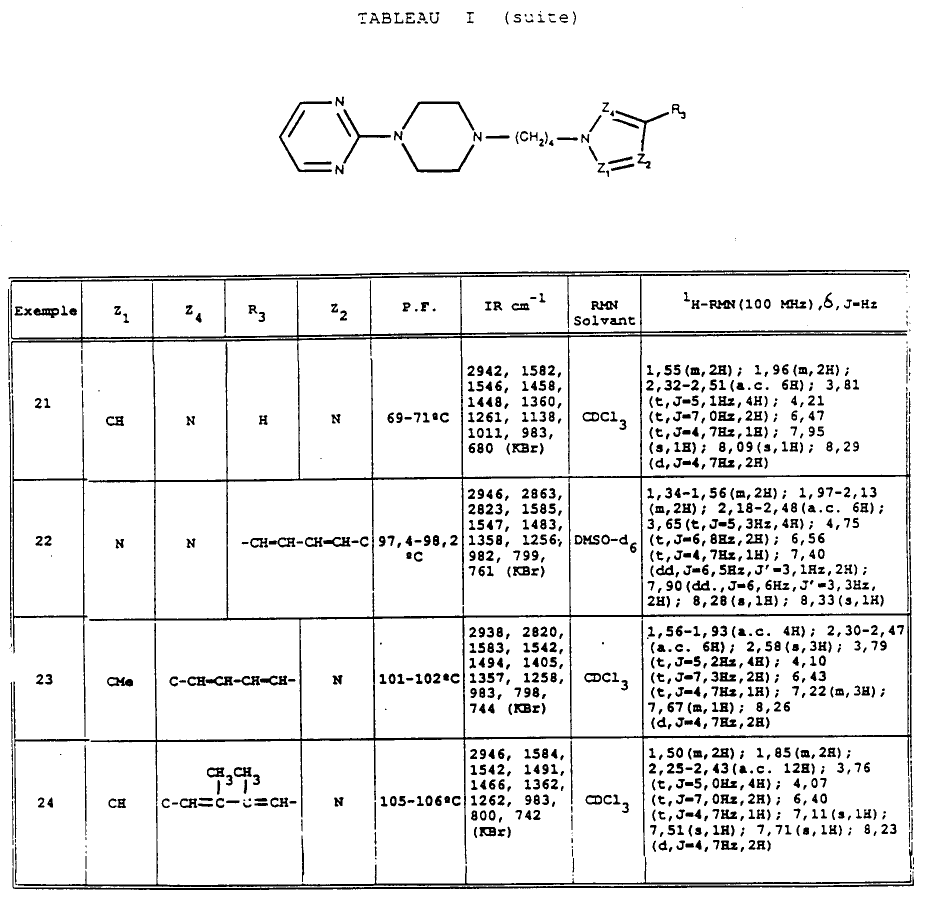

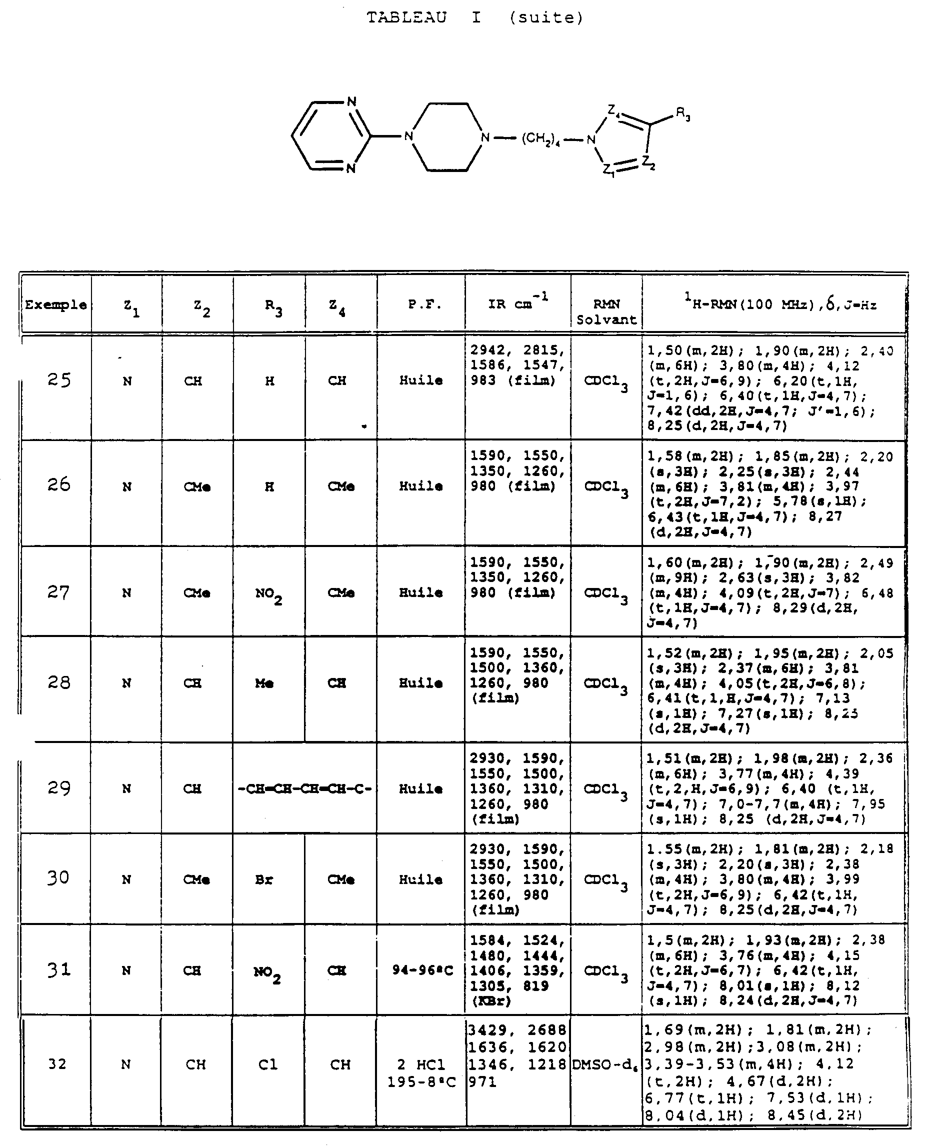

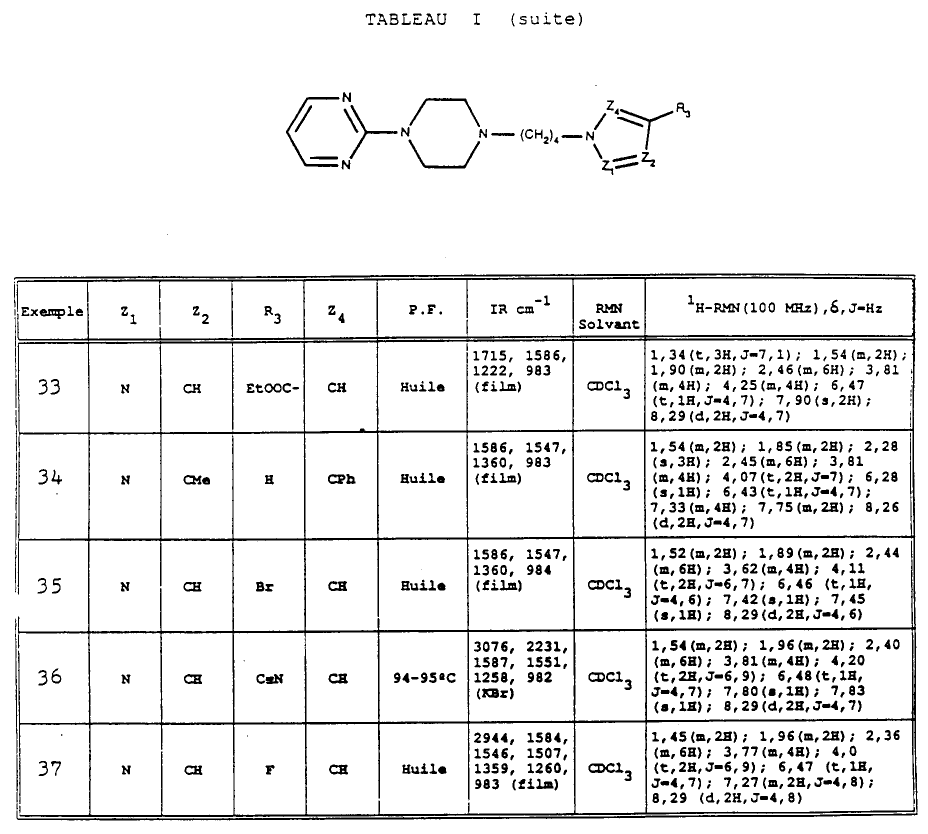

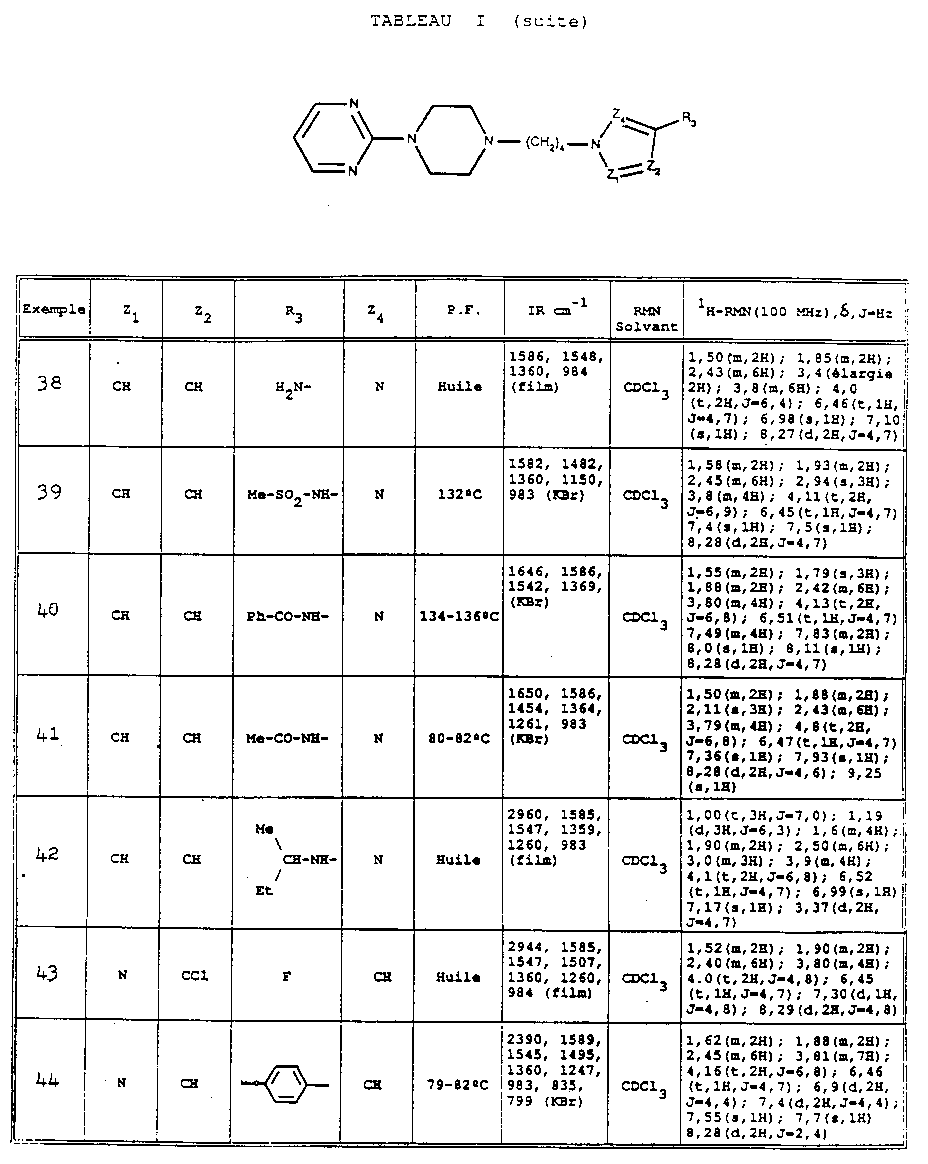

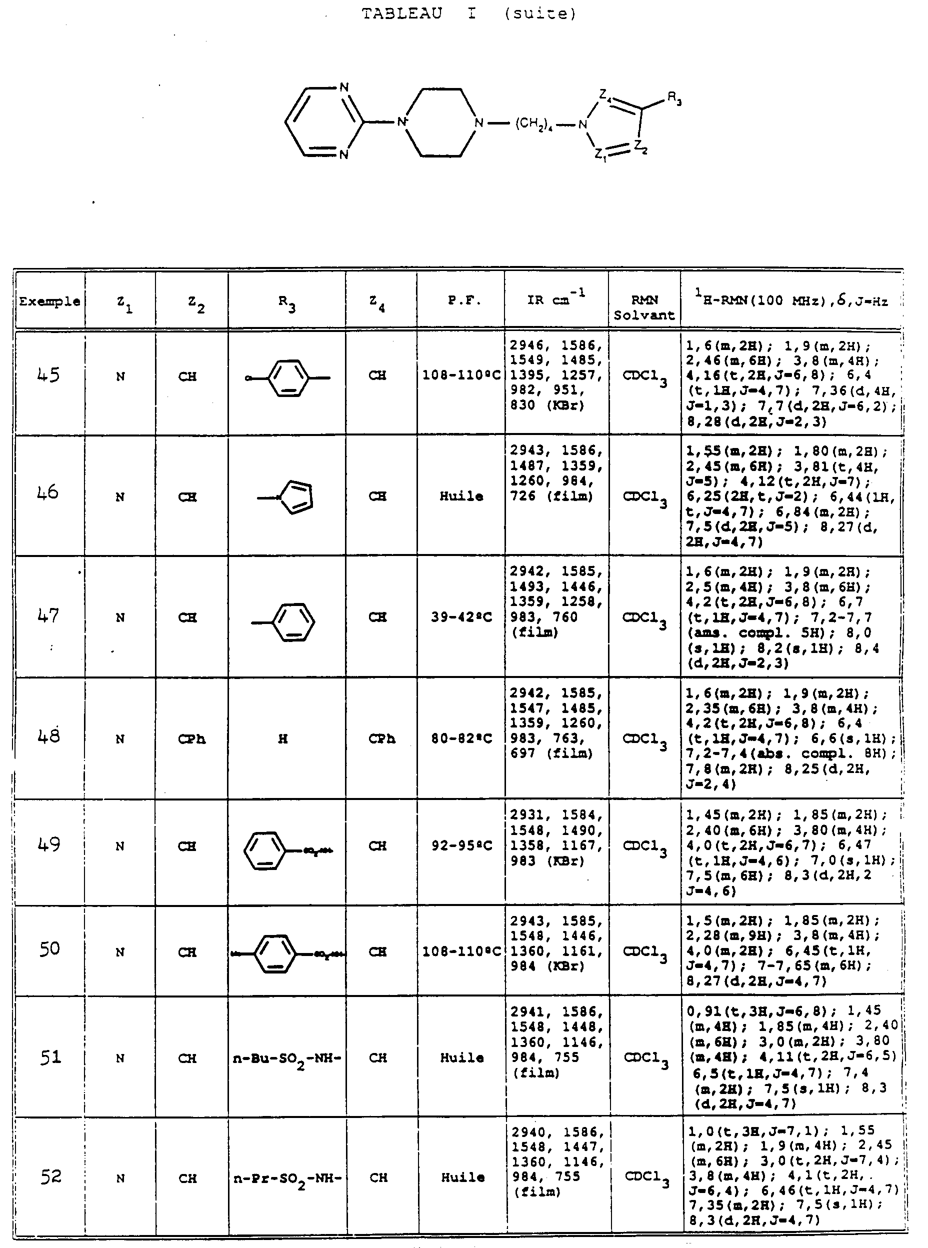

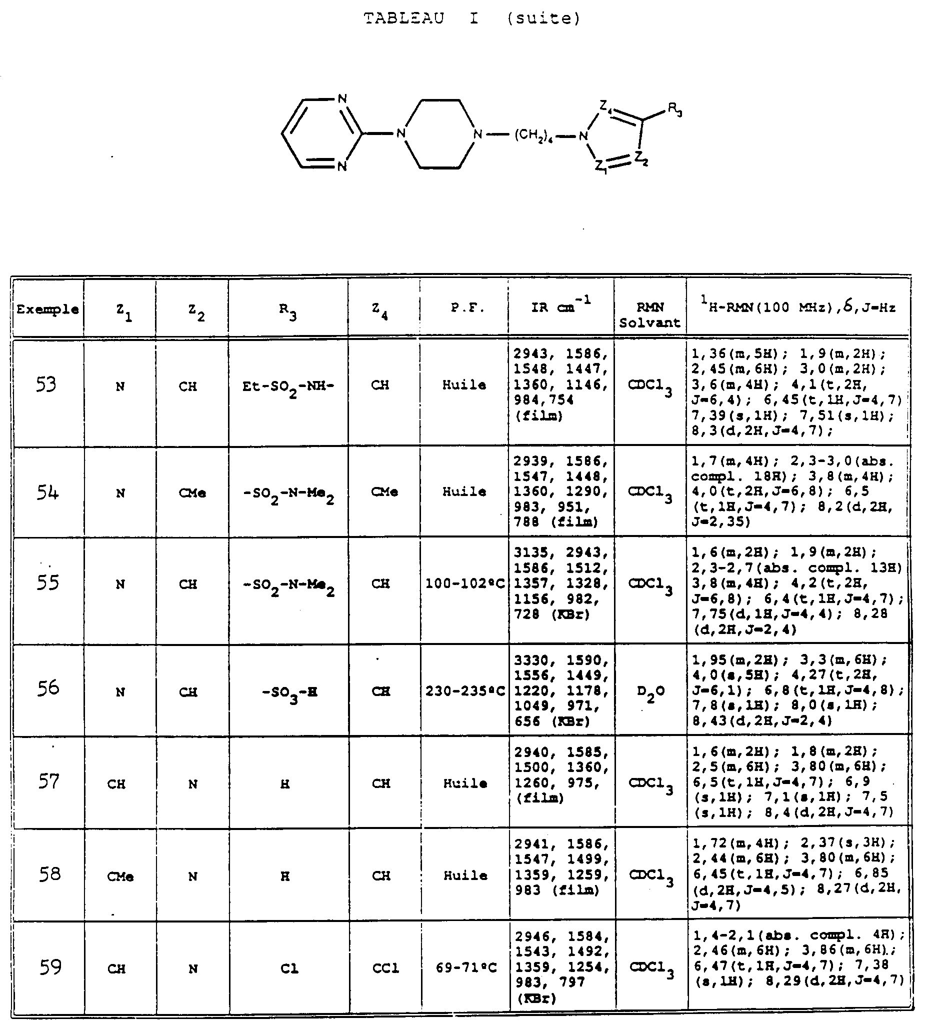

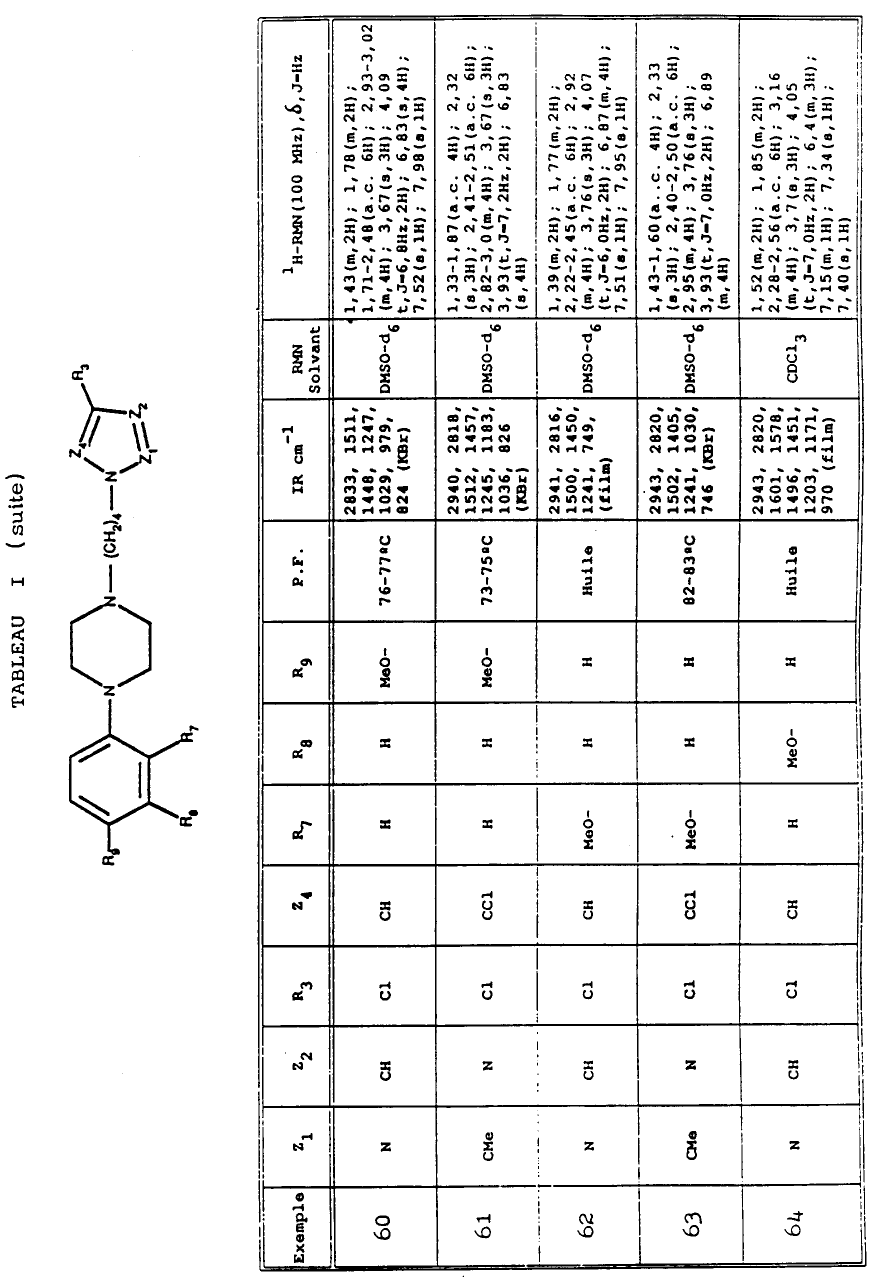

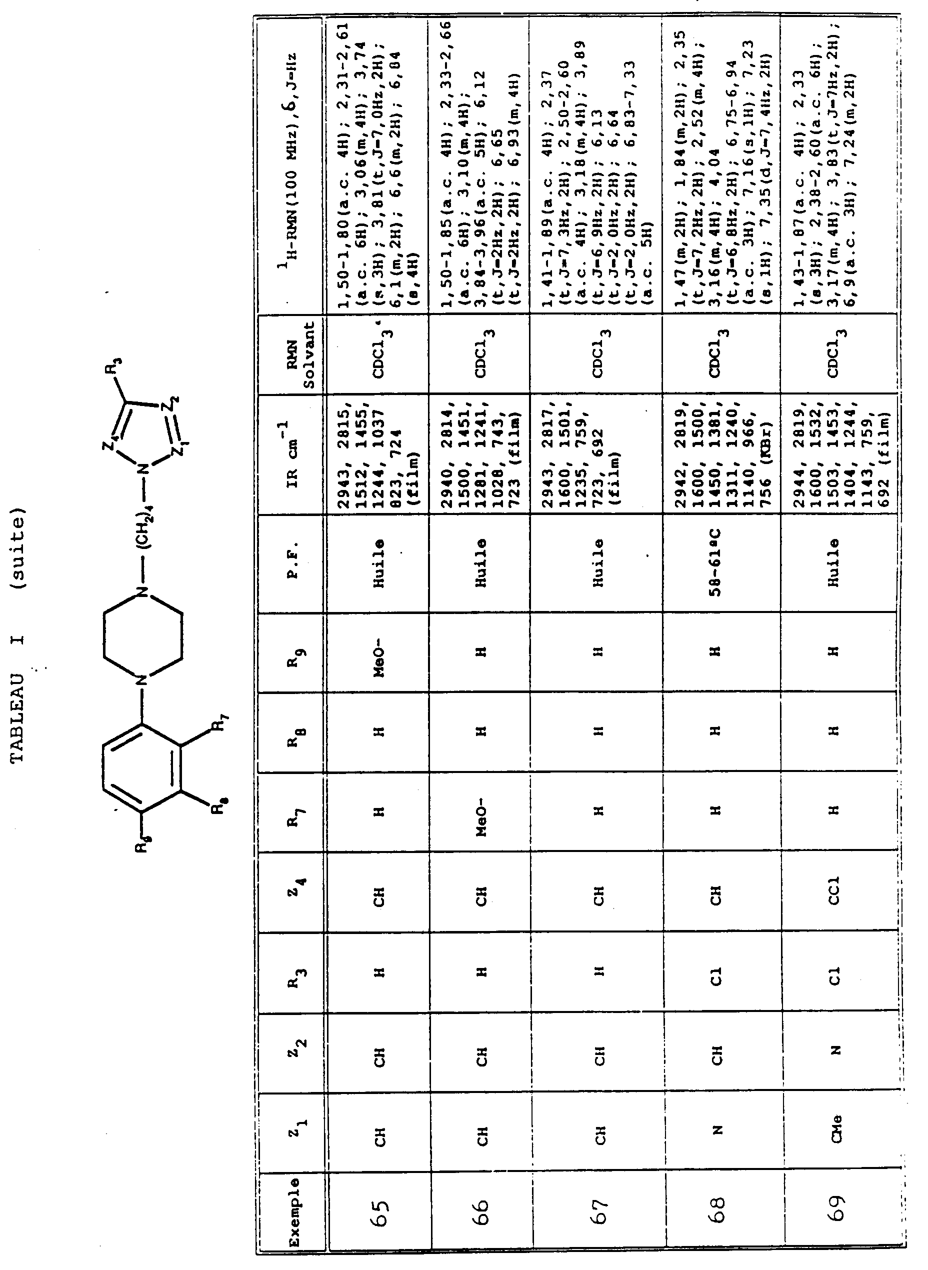

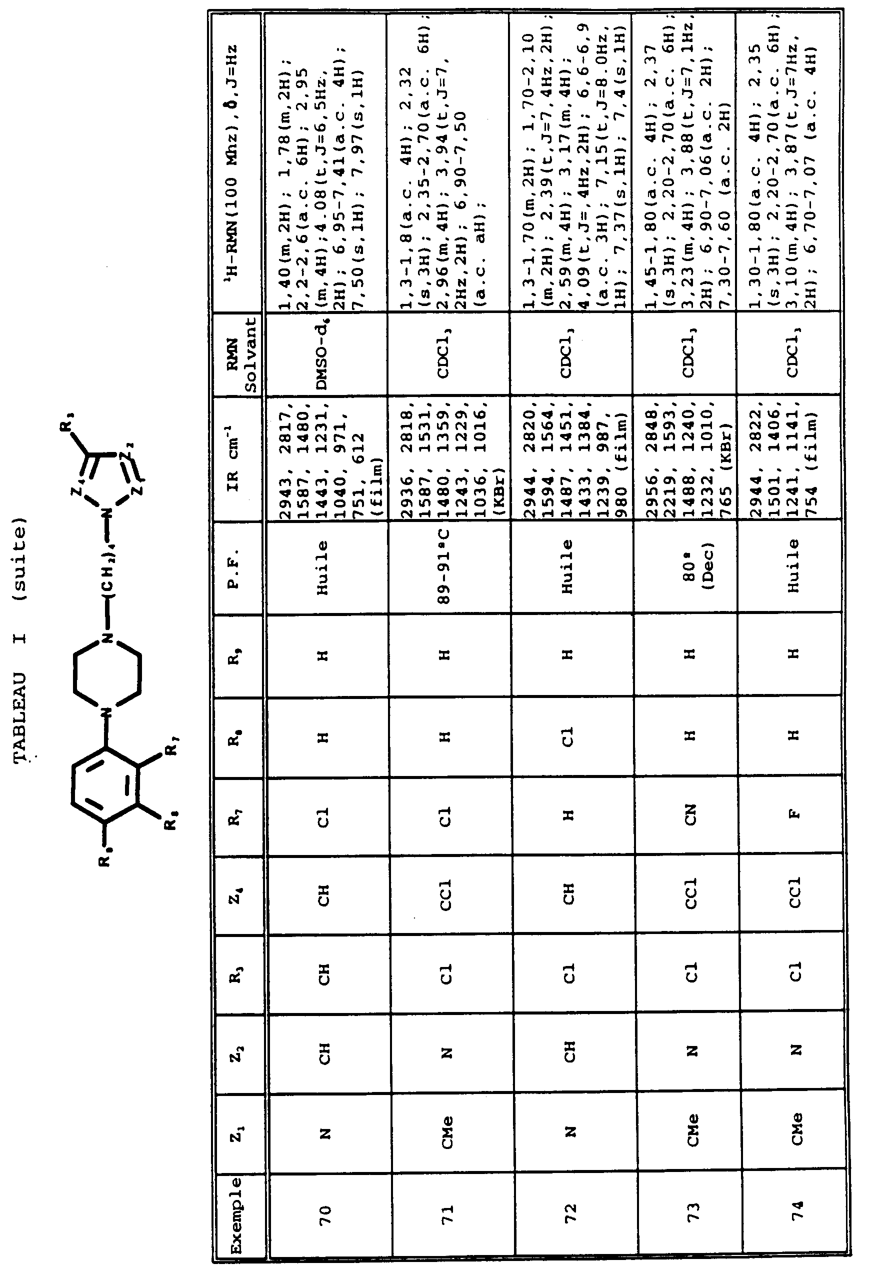

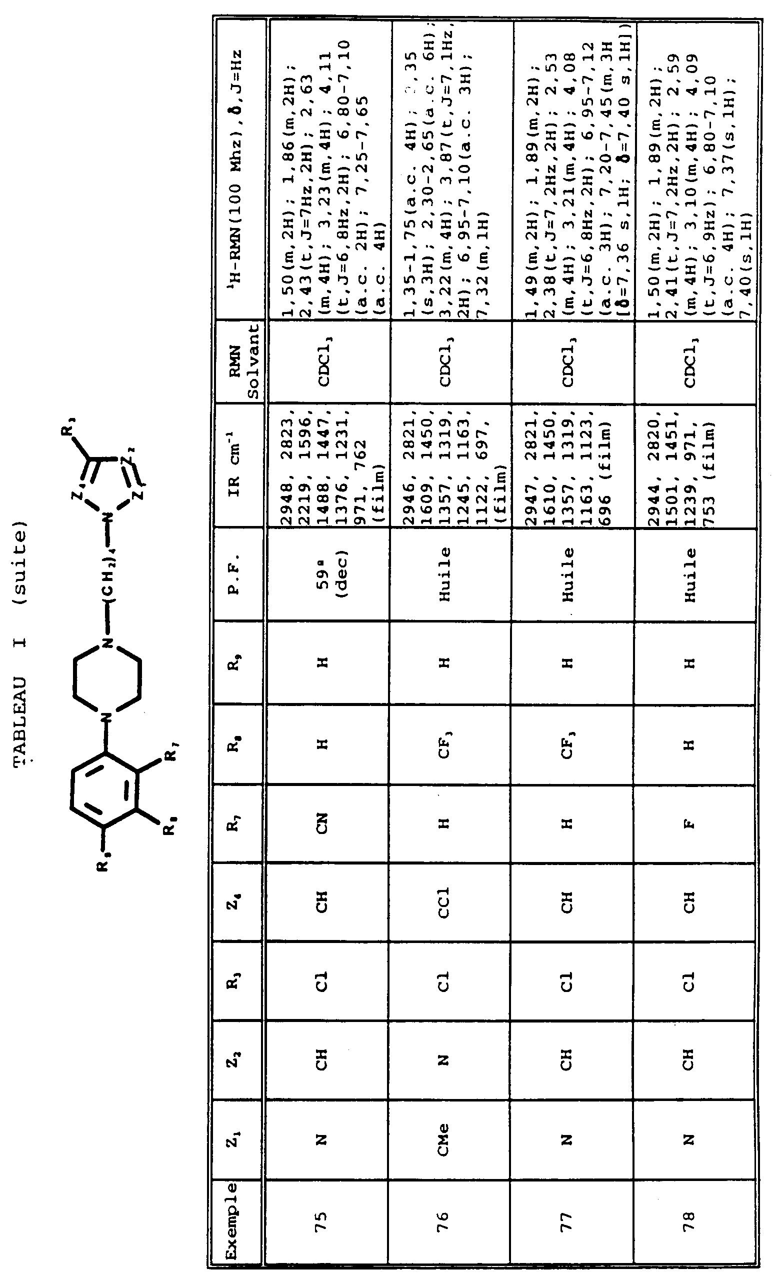

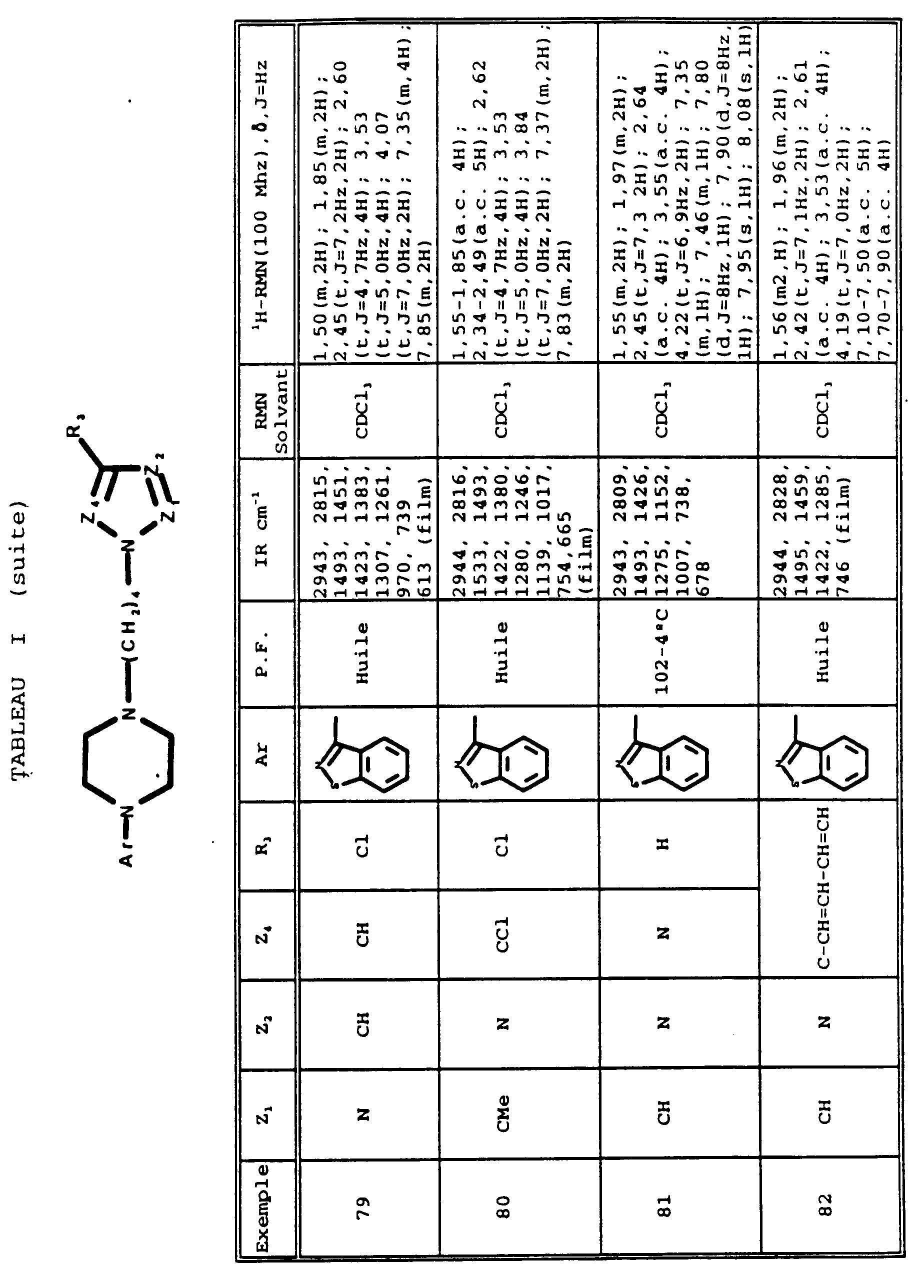

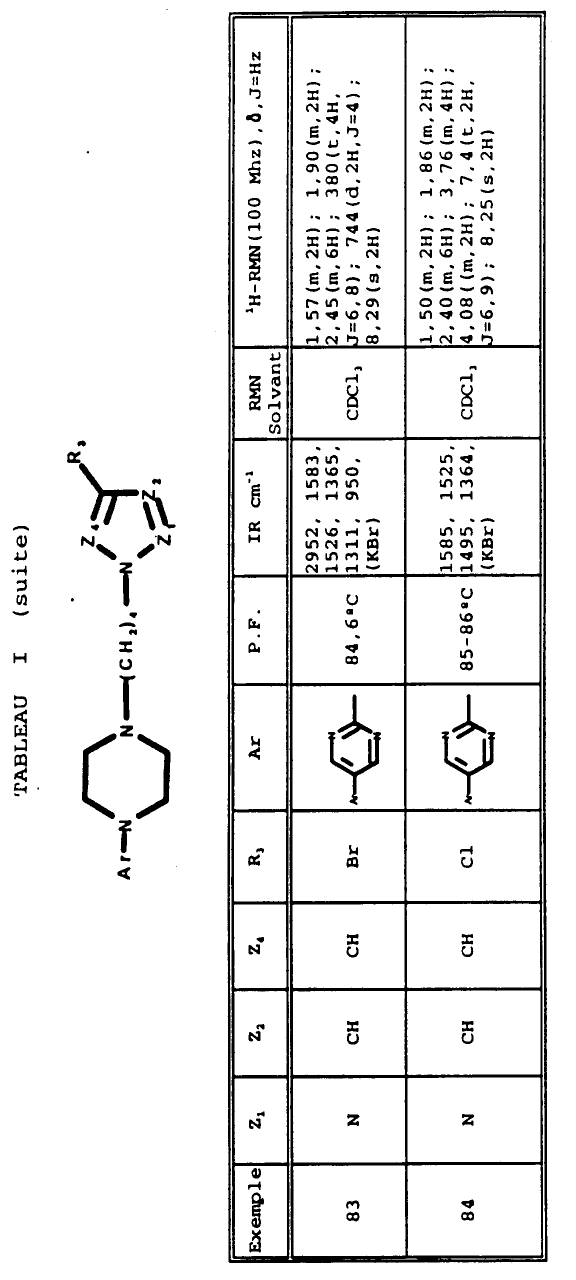

### Activité inhibitrice de la sécrétion acide gastrique, Méthode de Shay [Shay, H.; Komarov, S.A.; Fels, S.S.; Merange, D.; Grvenstein, M.; Siplet, H.: Gastroenterology, 5, 43 (1945) - Visscher, F.E.; Seay, P.H.; Taxelaar, A.P.; Veldhamp, W.; Vander Brook, M.J.: J. Pharmac. Exp. Ther., 110, 188 (1954) - "Animal Experiments in Pharmacological Analysis", F.R. Domer; C.C. Thomas Pub, Springfield, Illinois, USA, 1970, p. 140].

Dans ce test, on utilise des rats Wistar, mâles, de 200 à 250 grammes de poids, que l'on maintient à jeûn depuis le jour antérieur à celui de l'essai, avec libre accès à l'eau. On utilise des lots de 4 animaux chacun, au minimum.

On anesthésie les rats avec de l'éther éthylique, on leur pratique une laparotomie et on leur ligature le pylore, puis on effectue la ligature de l'incision abdominale. L'administration des produits, avec le véhicule pour le lot témoin, s'effectue par voie intraduodénale (i.d.) avant de ligature l'incision abdominale. La dose administrée pour le premier essai est de 40 mg/kg et on détermine également dans un deuxième essai la dose efficace cinquante (DE-50) par voie i.d.. Le véhicule utilisé est la gomme arabique à 5% p/v dans l'eau bidistillée.

Deux heures après la ligature du pylore, on sacrifie les rats par anesthésie prolongée avec de l'éther éthylique et on mesure le volume du suc gastrique, on détermine l'acidité totale moyennant un pH-mètre muni d'une burette automatique. Pour chaque produit et pour chaque dose essayés, on détermine le pourcentage d'inhibition de la sécrétion acide gastrique par rapport au lot témoin.

A titre d'exemples non limitatifs, on résume les résultats obtenus pour quelques-uns des dérivés de la présente invention dans le tableau II.

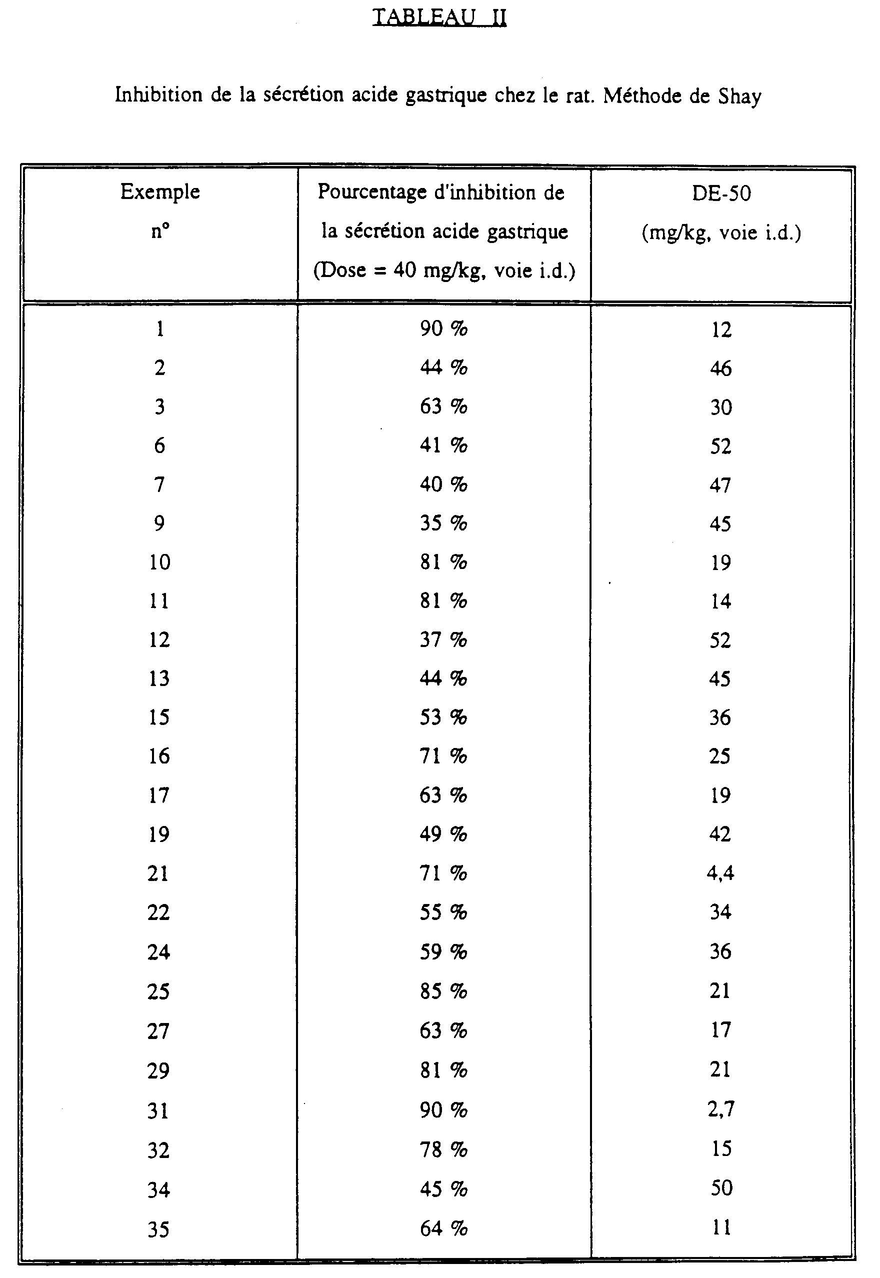

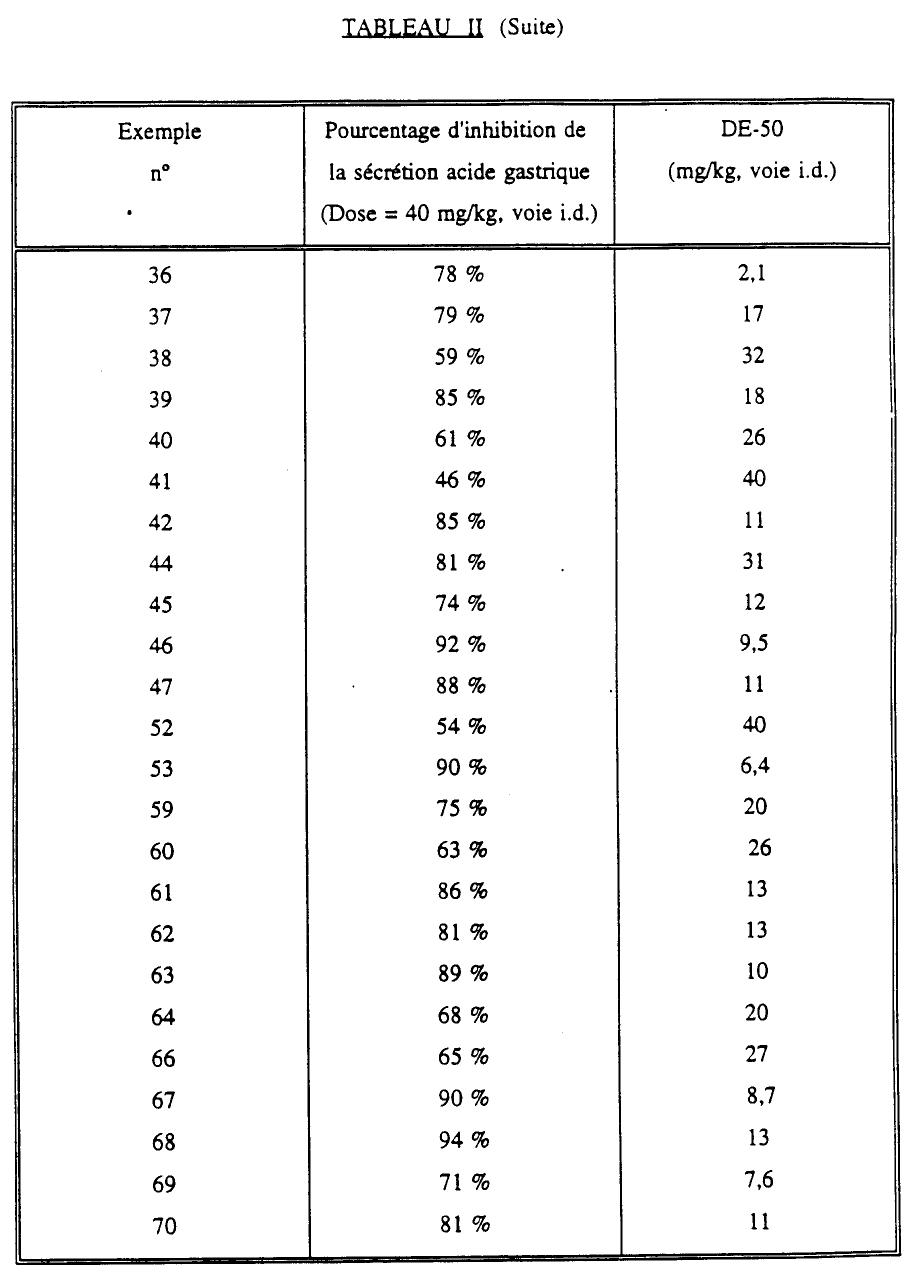

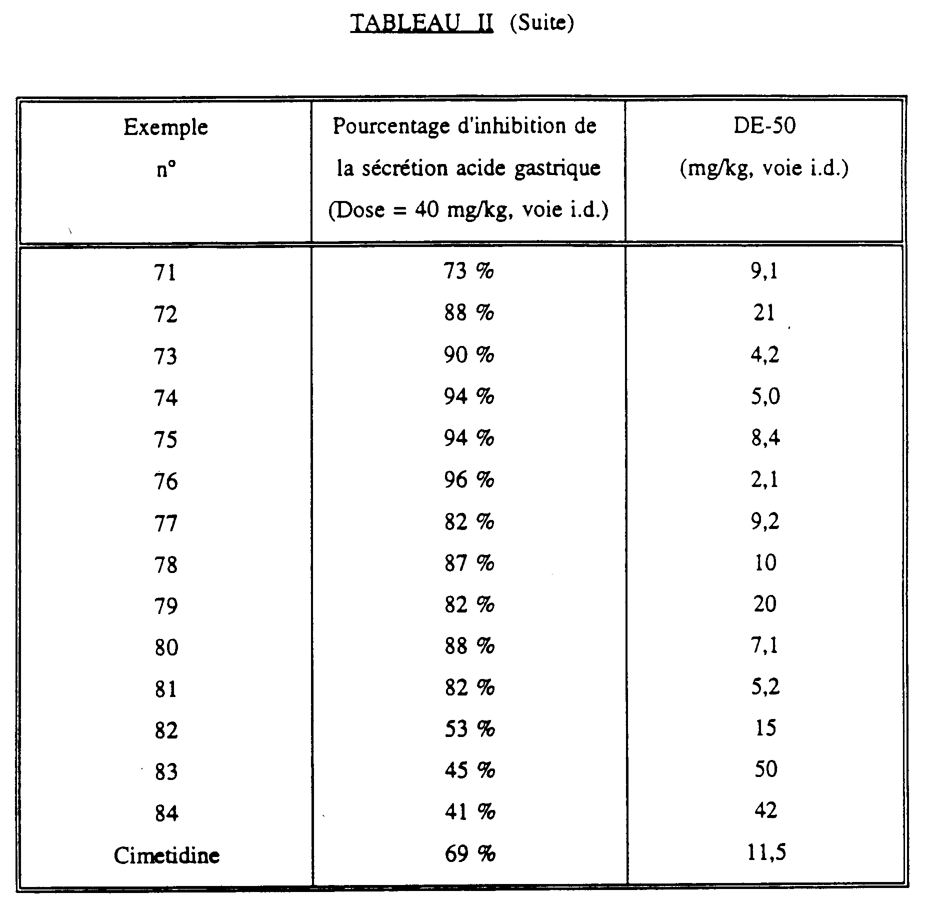

En thérapeutique humaine, la dose d'administration est bien sûr fonction de la gravité de l'affection à traiter. Elle sera généralement comprise entre environ 5 et environ 100 mg/jour. Les dérivés de l'invention seront, par exemples, administrés sous forme de comprimés, de solutions ou de suspension, ou bien de gélules. On indiquera, ci-après, à titre d'exemples, deux formes galéniques particulières.

| Exemple de formule par comprimé | |
|---|---|
| Composé de l'exemple 32 | 5 mg |
| Lactose | 60 mg |
| Cellulose microcristalline | 25 mg |
| Povidone | 5 mg |
| Amidon prégélatinisé | 3 mg |
| Dioxyde de silice colloïdale | 1 mg |
| Stéarate de magnésium | 1 mg |
| Poids comprimé | 100 mg |

| Exemple de formule par gélule | |
|---|---|
| Composé de l'exemple 32 | 10 mg |
| Glycérine polyoxyéthylénée | 135 mg |
| Behénate de glycérine | 5 mg |
| | 150 mg |

### Excipient : gélatine molle q.s.

Compte-tenu des intéressantes propriétés pharmacologiques attachées aux composés de formule générale I, la présente invention s'étend à l'application de ces composés à titre de médicaments, aux compositions pharmaceutiques les contenant et à leur utilisation pour la fabrication de médicaments destinés au traitement des maladies gastrointestinaies, en particulier pour la fabrication d'agents inhibiteurs de la sécrétion d'acide gastrique et d'agents antiulcéreux.

## Revendications

1. Utilisation des dérivés de formule générale I 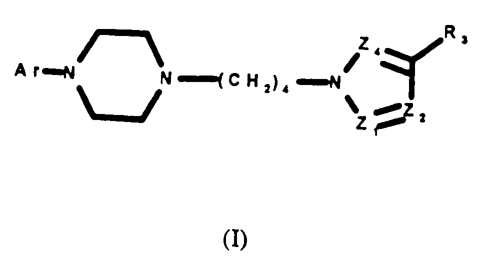 dans laquelle
Ar représente un radical aromatique azoté ou non, choisi parmi les aryles différemment substitués, la 2-pyrimidine différemment substituée, et le 3-(1,2-benzisothiazole).
Z₁ représente un atome d'azote ou un atome de carbone substitué ou non qu'on peut représenter par: C-R₁.
Z₂ représente un atome d'azote ou un atome de carbone substitué ou non qu'on peut représenter par: C-R₂.
Z₄ représente un atome d'azote ou un atome de carbone substitué ou non qu'on peut représenter par: C-R₄.
et R₁, R₂, R₃ et R₄, identiques ou différents, pouvant également former une partie d'un autre cycle, aromatique ou non, représentent un atome d'hydrogène, un halogène, un radical alkyle inférieur, un radical nitro, un radical hydroxy, un radical alcoxy, un radical cyano, un radical carboxylique, un radical carboxamido, un radical carboxylate d'alkyle, un radical aryle ou aryle substitué, un radical sulfonique, un radical sulfonamido, substitué ou non sur le groupement amino, un radical amino ou amino substitué,
et leurs sels thérapeutiquement acceptables, pour la fabrication de médicaments destinés au traitement des maladies gastro-intestinales, principalement indiqués comme inhibiteurs de la sécrétion d'acide gastrique ou comme agents antiulcéreux.

2. Utilisation selon la revendication 1, caractérisée en ce que le dérivé de formule générale I est choisi parmi :
1 - 1-{4-[4-(2-pyrimidinyl)-1-pipérazinyl]-butyl}-pyrrole,
2 - 1-{4-[4-(2-pyrimidinyl)-1-pipérazinyl]-butyl}-carbazole,
3 - 1-{4-[4-(2-pyrimidinyl)-1-pipérazinyl]-butyl}-indole,
4 - 2,3-diphényl-1-{4-[4-(2-pyrimidinyl)-1-pipérazinyl]-butyl}-indole,
5 - 4-carboxamido-1-{4-[4-(2-pyrimidinyl)-1-pipérazinyl]-butyl}-1*H*-pyrazole,
6 - 4-carboxy-1-{4-[4-(2-pyrimidinyl)-1-pipérazinyl]-butyl}-1*H*-pyrazole.
7 - 3-méthyl-5-trifluorométhyl-1-{4-[4-(2-pyrimidinyl)-1-pipérazinyl]-butyl}-1*H*-pyrazole,
8 - 4,5-diphényl-1-{4-[4(2-pyrimidinyl)-1-pipérazinyl]-butyl}-1*H*-imidazole,
9 - 2,4,5,-triphényl-1-{4-[4-(2-pyrimidinyl)-1-pypérazinyl]-butyl}-1*H*-imidazole,
10 - 4,5-diphényl-2-méthyl-1-{4-[4-(2-pyrimidinyl)-1-pipérazinyl]-butyl}-1*H*-imidazole,
11 - 4,5,-dichloro-2-méthyl-1-{4-[4-(2-pyrimidinyl)-1-pipérazinyl]-butyl}-1*H*-imidazole,
12 - 2-éthyl-1-{4-[4-(2-pyrimidinyl)-1-pipérazinyl]-butyl}-1*H*-imidazole,
13 - 2-phényl-1-{4-[4-(2-pyrimidinyl)-1-pipérazinyl]-butyl}-1*H*-imidazole,
14 - 4-carboxylatedeméthyle-1-{4-[4-(2-pyrimidinyl)-1-pipérazinyl]-butyl}-1*H*-imidazole,
15 - 4-phényl-1-{4-[4-(2-pyrimidinyl)-1-pipérazinyl]-butyl}-1*H*-imidazole,
16 - 1-{4-[4-(2-pyrimidinyl)-1-pipérazinyl]-butyl}-1*H*-benzimidazole,
17 - 1-{4-[4-(2-pyrimidinyl)-1-pipérazinyl]-butyl}-3*H*-imidazo[5,4-b]pyridine,
18 - 1-{4-[4-(2-pyrimidinyl)-1-pipérazinyl]-butyl}-1*H*-imidazo[4,5-b]pyridine,
19 - 1-{4-[4-(2-pyrimidinyl)-1-pipérazinyl]-butyl}-1*H*-benzotriazole,
20 - 2-chloro-1-{4-[4-(2-pyrimidinyl)-1-pipérazinyl]-butyl}-1*H*-benzimidazole,
21 - 1-{4-[4-(2-pyrimidinyl)-1-pipérazinyl]-butyl}-1*H*-1,2,4-triazole,
22 - 2-{4-[4-(2-pyrimidinyl)-1-pipérazinyl]-butyl}-2*H*-benzotriazole,
23 - 2-méthyl-1-{4-[4-(2-pyrimidinyl)-1-pipérazinyl]-butyl}-1*H*-benzimidazole,
24- 5,6-diméthyl-1-{4-[4-(2-pyrimidinyl)-1-pipérazinyl]-butyl}-1*H*-benzimidazole,
25 - 1-{4-[4-(2-pyrimidinyl)-1-pipérazinyl]-butyl}-1*H*-pyrazole,
26 - 3,5-diméthyl-1-{4-[4-(2-pyrimidinyl)-1-pipérazinyl]-butyl}-1*H*-pyrazole,
27 - 3,5-diméthyl-4-nitro-1-{4-[4-(2-pyrimidinyl)-1-pipérazinyl]-butyl}-1*H*-pyrazole,
28 - 4-méthyl-1-{4-[4-(2-pyrimidinyl)-1-pipérazinyl]-butyl}-1*H*-pyrazole,
29 - 1-{4-[4-(2-pyrimidinyl)-1-pipérazinyl]-butyl}-1*H*-indazole,
30 - 4-bromo-3,5-diméthyl-1-{4-[4-(2-pyrimidinyl)-1-pipérazinyl]-butyl)-1*H*-pyrazole,
31 - 4-nitro-1-{4-[4-(2-pyrimidinyl)-1-pipérazinyl]-butyl}-1*H*-pyrazole,
32 - 4-chloro-1-{4-[4-(2-pyrimidinyl)-1-pipérazinyl]-butyl}-1*H*-pyrazole dichlorhydrate,
33 - 4-carboxylated'éthyle-1-{4-[4-(2-pyrimidinyl)-1-pipérazinyl]-butyl}-1*H*-pyrazole,
34 - 3-méthyl-5-phényl-1-{4-[4-(2-pyrimidinyl)-1-pipérazinyl]-butyl}-1*H*-pyrazole,
35 - 4-bromo-1-{4-[4-(2-pyrimidinyl)-1-pipérazinyl]-butyl}-1*H*-pyrazole,
36 - 4-cyano-1-{4-[4-(2-pyrimidinyl)-1-pipérazinyl]-butyl}-1*H*-pyrazole,
37 - 4-fluoro-1-{4-[4-(2-pyrimidinyl)-1-pipérazinyl]-butyl}-1*H*-pyrazole,
38 - 4-amino-1-{4-[4-(2-pyrimidinyl)-1-pipérazinyl]-butyl}-1*H*-pyrazole,
39 - 4-méthylsulfonamido-1-{4-[4-(2-pyrimidinyl)-1-pipérazinyl]-butyl}-1*H*-pyrazole,
40 - 4-benzamido-1-{4-[4-(2-pyrimidinyl)-1-pipérazinyl]-butyl}-1H-pyrazole,
41 - 4-acétamido-1-{4-[4-(2-pyrimidinyl)-1-pipérazinyl]-butyl}-1*H*-pyrazole,
42 - 4-(2-butyl)amino-1-{4-[4-(2-pyrimidinyl)-1-pipérazinyl]-butyl}-1*H*-pyrazole,
43 - 3-chloro-4-fluoro-1-{4-[4-(2-pyrimidinyl)-1-pipérazinyl]-butyl)-1*H*-pyrazole,
44 - 4-(4-méthoxyphényl)-1-{4-[4-(2-pyrimidinyl)-1-pipérazinyl]-butyl}-1*H*-pyrazole,
45 - 4-(4-méthoxyphényl)-1-{4-[4-(2-pyrimidinyl)-1-pipérazinyl]-butyl}-1*H*-pyrazole,
46 - 4-(1-pyrrolyl)-1-{4-[4-(2-pyrimidinyl)-pipérazinyl]-butyl}-1*H*-pyrazole,
47 - 4-phényl-1-{4-[4-(2-pyrimidinyl)-1-pipérazinyl]-butyl}-1*H*-pyrazole,
48 - 3,5-diphényl-1-{4-[4-(2-pyrimidinyl)-1-pipérazinyl]-butyl}-1*H*-pyrazole,
49 - 4-phénylsulfamoyl-1-{4-[4-(2-pyrimidinyl)-1-pipérazinyl]-butyl}1*H*-pyrazole,
50 - 4-(4-méthylbenzene)sulfamoyl-1-{4-[4-(2-pyrimidinyl)-1-pipérazinyl]-butyl}-1*H*-pyrazole,
51 - 4-butylsulfamoyl-1-{4-[4-(2-pyrimidinyl)-1-pipérazinyl]-butyl}-1*H*-pyrazole,
52 - 4-propylsulfamoyl-1-{4-[4-(2-pyrimidinyl)-1-pipérazinyl]-butyl}-1*H*-pyrazole,
53 - 4-éthylsulfarnoyl-1-{4-[4-(2-pyrimidinyl)-1-pipérazinyl]-butyl}-1*H*-pyrazole,
54 - 3,5-diméthyl-(N,N-diméthylsulfonamido)-1-{4-[4-(2-pyrimidinyl)-1-pipérazinyl] butyl}-1*H*-pyrazole,
55 - 4-N-méthylsulfamoyl-1-{4-[4-(2-pyrimidinyl)-1-pipérazinyl]-butyl}-1*H*-pyrazole,
56 - 4-sulfonique-1-{4-[4-(2-pyrimidinyl)-1-pipérazinyl]-butyl}-1*H*-pyrazole,
57 - 1-{4-[4-(2-pyrimidinyl)-1-pipérazinyl]-butyl}-1*H*-imidazole,
58 - 2-méthyl-1-{4-[4-(2-pyrimidinyl)-1-pipérazinyl]-butyl}-1*H*-imidazole,
59 - 4,5-dichloro-1-{4-[4-(2-pyrimidinyl)-1-pipérazinyl]-butyl}-1*H*-imidazole,
60 - 4-chloro-1- {4-[4-(4-méthoxyphényl)-1-pipérazinyl]-butyl}-1*H*-pyrazole,
61 - 4,5-dichloro-2-méthyl-1-{4-[4-(4-méthoxyphényl)-1-pipérazinyl]-butyl}- 1*H*-imidazole,
62 - 4-chloro-1-{4-[4-(2-méthoxyphényl)-1-pipérazinyl]-butyl}-1*H*-pyrazole,
63 - 4,5-dichloro-2-méthyl-1-{4-[4-(2-méthoxyphényl)-1-pipérazinyl]-butyl}-1*H*-imidazole,
64 - 4-chloro-1-{4-[4-(3-méthoxyphényl)-1-pipérazinyl]-butyl)-1*H*-pyrazole,
65 - 1-{4-[4-(4-méthoxyphényl)-1-pipérazinyl]-butyl}-pyrrole,
66 - 1-{4-[4-(2-méthoxyphényl)-1-pipérazinyl]-butyl}-pyrrole,
67 - 1-{4-[4(phényl)-1-pipérazinyl]-butyl}-pyrrole,
68 - 4-chloro-1-{4-[4-(phényl)-1-pipérazinyl]-butyl}-1*H*-pyrazole,
69 - 4,5-dichloro-2-méthyl-1-{4-[4-(phényl)-1-pipérazinyl]-butyl}-1*H*-imidazole,
70 - 4-chloro-1-{4-[4-(2-chlorophényl)-1-pipérazinyl]-butyl}-1*H*-pyrazole,
71 - 4,5-dichloro-2-méthyl-1-{4-[4-(2-chlorophényl)-1-pipérazinyl]-butyl}-1*H*-imidazole,
72 - 4-chloro-1-{4-[4-(3-chlorophényl)-1-pipérazinyl]-butyl}-1*H*-pyrazole,
73 - 4,5-dichloro-2-méthyl-1-{4-[4-(2-cyanophényl-1-pipérazinyl]-butyl}-1*H*-imidazole,
74 - 4,5-dichloro-2-méthyl-1-{4-[4-(2-fluorophényl)-1-pipérazinyl]-butyl}-1*H*-imidazole,
75 - 4-chloro-1-{4-[4-(2-cyanophényl)-1-pipérazinyl]-butyl}-1*H*-pyrazole,
76 - 4,5-dichloro-2-méthyl-1-{4-[4-(3-trifluorométhylphényl)-1-pipérazinyl]-butyl}-1*H*-imidazole,
77 - 4-chloro-1-{4-[4-(3-trifluorométhylphényl)-1-pipérazinyl]-butyl}-1*H*-pyrazole,
78 - 4-chloro-1-{4-[4-(2-fluorophényl)-1-pipérazintyl]-butyl}-1*H*-pyrazole,
79 - 4-chloro-1-{4-[4-(1,2-benzisothiazol-3-yl)-1-pipérazinyl]butyl}-1*H*-pyrazole,
80 - 4,5-dichloro-2-méthyl-1-{4-[4-(1,2-benzisothiazol-3-yl)-1-pipérazinyl]-butyl}-1*H*-imidazole,
81 - 1-{4-[4-(1,2-benzisothiazol-3-yl)-1 -pipérazinyl]-butyl}-1*H*-1,2,4-triazole,
82 - 1-{4-[4-(1,2-benzisothiazol-3-yl)-1-pipérazinyl]-butyl}-1*H*-benzimidazole,
83 - 4-bromo-1-{4-[4-(5-bromopyrimidin-2-yl)-1-pipérazinyl]-butyl}-1*H*-pyrazole,
84 - 4-bromo-1-{4-[4-(5-chloropyrimidin-2-yl)-1-pipérazinyl]-butyl}-1*H*-pyrazole,

## Claims

1. Use of the derivatives of general formula I 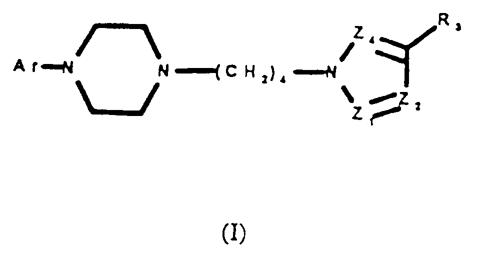 in which
Ar represents an optionally nitrogenous aromatic radical chosen from the variously substituted aryls, variously substituted 2-pyrimidine and 3-(1,2-benzisothiazole),
Z₁ represents a nitrogen atom or an optionally substituted carbon atom which can be represented by: C-R₁,
Z₂, represents a nitrogen atom or an optionally substituted carbon atom which can be represented by: C-R₂,
Z₄ represents a nitrogen atom or an optionally substituted carbon atom which can be represented by:C-R₄,
and R₁, R₂, R₃ and R₄, which are identical or different and which can also form part of another, optionally aromatic, ring, represent a hydrogen atom, a halogen, a lower alkyl radical, a nitro radical, a hydroxyl radical, an alkoxy radical, a cyano radical, a carboxyl radical, a carboxamido radical, an alkyl carboxylate radical, an aryl or substituted aryl radical, a sulpho radical, a sulphonamido radical which is optionally substituted on the amino group, or an amino or substituted amino radical,
and their therapeutically acceptable salts for the manufacture of medicaments intended for the treatment of gastrointestinal diseases, which are mainly indicated as inhibitors of gastric acid secretion or as anti-ulcer agents.

2. Use according to Claim 1, characterized in that the derivative of general formula I is chosen from :
1 - 1-{4-[4-(2-pyrimidinyl)-1-piperazinyl]butyl}pyrrole,
2 - 1-{4-[4-(2-pyrimidinyl)-1-piperazinyl]butyl}carbazole,
3 - 1-{4-[4-(2-pyrimidinyl)-1-piperazinyl]butyl}indole,
4 - 2,3-diphenyl-1-{4-[4-(2-pyrimidinyl)-1-piperazinyl]-butyl}indole,
5 - 4-carboxamido-1-{4-[4-(2-pyrimidinyl)-1-piperazinyl]butyl}-1*H*-pyrazole,
6 - 4-carboxy-1-{4-(4-(2-pyrimidinyl)-1-piperazinyl]-butyl}-1*H*-pyrazole,
7 - 3-methyl-5-trifluoromethyl-1-{4-[4-(2-pyrimidinyl)-1-piperazinyl]butyl}-1*H*-pyrazole,
8 - 4,5-diphenyl-1-{4-[4-(2-pyrimidinyl)-1-piperazinyl] butyl}-1*H*-imidazole,
9 - 2,4,5-triphenyl-1-{4-[4-(2-pyrimidinyl)-1-piperazinyl]butyl}-1*H*-imidazole,
10 - 4,5-diphenyl-2-methyl-1-{4-[4-(2-pyrimidinyl)-1-piperazinyl]butyl}-1*H*-imidazole,
11 - 4,5-dichloro-2-methyl-1-{4-[4-(2-pyrimidinyl)-1-piperazinyl]butyl}-1*H*-imidazole,
12 - 2-ethyl-1-{4-(4-(2-pyrimidinyl)-1-piperazinyl]-butyl}-1*H*-imidazole,
13 - 2-phenyl-1-{4-[4-(2-pyrimidinyl)-1-piperazinyl]-butyl}-1*H*-imidazole,
14 - 4-methoxycarbonyl-1-{4-[4-(2-pyrimidinyl)-1-piperazinyl]butyl}-1*H*-imidazole,
15 - 4-phenyl-1-{4-[4-(2-pyrimidinyl)-1-piperazinyl]-butyl}-1*H*-imidazole,
16 - 1-{4-[4-(2-pyrimidinyl)-1-piperazinyl]butyl}-1*H*-benzimidazole,
17 - 1-{4-[4-(2-pyrimidinyl)-1-piperazinyl]butyl}-1*H*-imidazo[5,4-b]pyridine,
18 - 1-{4-[4-(2-pyrimidinyl)-1-piperazinyl]butyl}-1*H*-imidazo[4,5-b]pyridine,
19 - 1-{4-[4-(2-pyrimidinyl)-1-piperazinyl]butyl}-1*H*-benzotriazole,
20 - 2-chloro-1-{4-[4-(2-pyrimidinyl)-1-piperazinyl]-butyl}-1*H*-benzimidazole,
21 - 1-{4-[4-(2-pyrimidinyl)-1-piperazinyl]butyl}-1*H*-1,2,4-triazole,
22 - 2-{4-[4-(2-pyrimidinyl)-1-piperazinyl]butyl}-2*H*-benzotriazole,
23 - 2-methyl-1-{4-[4-(2-pyrimidinyl)-1-piperazinyl]-butyl}-1*H*-benzimidazole,
24 - 5,6-dimethyl-1-{4-[4-(2-pyrimidinyl)-1-piperazinyl]-butyl}-1*H*-benzimidazole,
25 - 1-{4-[4-(2-pyrimidinyl)-1-piperazinyl]butyl}-1*H*-pyrazole,
26 - 3,5-dimethyl-1-{4-[4-(2-pyrimidinyl)-1-piperazinyl]-butyl}-1*H*-pyrazole,
27 - 3,5-dimethyl-4-nitro-1-{4-[4-(2-pyrimidinyl)-1-piperazinyl]butyl}-1*H*-pyrazole,
28 - 4-methyl-1-{4-[4-(2-pyrimidinyl)-1-piperazinyl]-butyl}-1*H*-pyrazole,
29 - 1-{4-[4-(2-pyrimidinyl)-1-piperazinyl]butyl}-1*H*-indazole,
30 - 4-bromo-3,5-dimethyl-1-{4-[4-(2-pyrimidinyl)-1-piperazinyl]butyl}-1*H*-pyrazole,
31 - 4-nitro-1-{4-[4-(2-pyrimidinyl)-1-piperazinyl]-butyl}-1*H*-pyrazole,
32 - 4-chloro-1-{4-[4-(2-pyrimidinyl)-1-piperazinyl]-butyl}-1*H*-pyrazole dihydrochloride,
33 - 4-ethoxycarbonyl-1-{4-[4-(2-pyrimidinyl)-1-piperazinyl]butyl}-1*H*-pyrazole,
34 - 3-methyl-5-phenyl-1-{4-[4-(2-pyrimidinyl)-1-piperazinyl]butyl}-1*H*-pyrazole,
35 - 4-bromo-1-{4-[4-(2-pyrimidinyl)-1-piperazinyl]-butyl}-1*H*-pyrazole,
36 - 4-cyano-1-{4-[4-(2-pyrimidinyl)-1-piperazinyl]-butyl}-1*H*-pyrazole,
37 - 4-fluoro-1-{4-[4-(2-pyrimidinyl)-1-piperazinyl]-butyl}-1*H*-pyrazole,
38 - 4-amino-1-{4-[4-(2-pyrimidinyl)-1-piperazinyl]-butyl}-1*H*-pyrazole,
39 - 4-methylsulphonamido-1-{4-[4-(2-pyrimidinyl)-1-piperazinyl]butyl}-1*H*-pyrazole,
40 - 4-benzamido-1-{4-[4-(2-pyrimidinyl)-1-piperazinyl]-butyl}-1*H*-pyrazole,
41 - 4-acetamido-1-{4-[4-(2-pyrimidinyl)-1-piperazinyl]-butyl}-1*H*-pyrazole,
42 - 4-(2-butyl)amino-1-{4-[4-(2-pyrimidinyl)-1-piperazinyl]butyl}-1*H*-pyrazole,
43 - 3-chloro-4-fluoro-1-{4-[4-(2-pyrimidinyl)-1-piperazinyl]butyl}-1*H*-pyrazole,
44 - 4-(4-methoxyphenyl)-1-{4-[4-(2-pyrimidinyl)-1-piperazinyl]butyl}-1*H*-pyrazole,
45 - 4-(4-chlorophenyl)-1-{4-[4-(2-pyrimidinyl)-1-piperazinyl]butyl}-1*H*-pyrazole,
46 - 4-(1-pyrrolyl)-1-{4-[4-(2-pyrimidinyl)-1-piperazinyl]butyl}-1*H*-pyrazole,
47 - 4-phenyl-1-{4-[4-(2-pyrimidinyl)-1-piperazinyl]-butyl}-1*H*-pyrazole,
48 - 3,5-diphenyl-1-{4-[4-(2-pyrimidinyl)-1-piperazinyl]-butyl}-1*H*-pyrazole,
49 - 4-phenylsulphamoyl-1-{4-[4-(2-pyrimidinyl)-1-piperazinyl]butyl}-1*H*-pyrazole,
50 - 4-(4-methylbenzene)sulphamoyl-1-{4-[4-(2-pyrimidinyl)-1-piperazinyl]butyl}-1*H*-pyrazole,
51 - 4-butylsulphamoyl-1-{4-[4-(2-pyrimidinyl)-1-piperazinyl]butyl}-1*H*-pyrazole,
52 - 4-propylsulphamoyl-1-{4-[4-(2-pyrimidinyl)-1-piperazinyl]butyl}-1*H*-pyrazole,
53 - 4-ethylsulphamoyl-1-{4-[4-(2-pyrimidinyl)-1-piperazinyl]butyl}-1*H*-pyrazole,
54 - 3,5-dimethyl-4-(N,N-dimethylsulphamoyl)-1-{4-[4-(2-pyrimidinyl)-1-piperazinyl]butyl}-1*H*-pyrazole,
55 - 4-N-methylsulphamoyl-1-{4-[4-(2-pyrimidinyl)-1-piperazinyl]butyl}-1*H*-pyrazole,
56 - 4-sulpho-1-{4-[4-(2-pyrimidinyl)-1-piperazinyl]-butyl}-1*H*-pyrazole,
57 - 1-{4-[4-(2-pyrimidinyl)-1-piperazinyl]butyl}-1*H-*imidazole,
58 - 2-methyl-1-{4-[4-(2-pyrimidinyl)-1-piperazinyl]-butyl}-1*H*-imidazole,
59 - 4,5-dichloro-1-{4-[4-(2-pyrimidinyl)-1-piperazinyl]-butyl}-1*H*-imidazole,
60 - 4-chloro-1-{4-[4-(4-methoxyphenyl)-1-piperazinyl]-butyl}-1*H*-pyrazole,
61 - 4,5-dichloro-2-methyl-1-{4-[4-(4-methoxyphenyl)-1-piperazinyl]butyl}-1*H*-imidazole,
62 - 4-chloro-1-{4-[4-(2-methoxyphenyl)-1-piperazinyl]-butyl}-1*H*-pyrazole,
63 - 4,5-dichloro-2-methyl-1-{4-[4-(2-methoxyphenyl)-1-piperazinyl]butyl}-1*H*-imidazole,
64 - 4-chloro-1-{4-[4-(3-methoxyphenyl)-1-piperazinyl]-butyl}-1*H*-pyrazole,
65 - 1-{4-[4-(4-methoxyphenyl)-1-piperazinyl]butyl}-pyrrole,
66 - 1-{4-[4-(2-methoxyphenyl)-1-piperazinyl]butyl}-pyrrole,
67 - 1-{4-[4-phenyl-1-piperazinyl]butyl}pyrrole,
68 - 4-chloro-1-{4-[4-phenyl-1-piperazinyl]butyl}-1*H*-pyrazole,
69 - 4,5-dichloro-2-methyl-1-{4-[4-phenyl-1-piperazinyl]-butyl}-1*H*-imidazole,
70 - 4-chloro-1-{4-[4-(2-chlorophenyl)-1-piperazinyl]-butyl}-1*H*-pyrazole,
71 - 4,5-dichloro-2-methyl-1-{4-[4-(2-chlorophenyl)-1-piperazinyl]butyl}-1*H*-imidazole,
72 - 4-chloro-1-{4-[4-(3-chlorophenyl)-1-piperazinyl]-butyl}-1*H*-pyrazole,
73 - 4,5-dichloro-2-methyl-1-{4-[4-(2-cyanophenyl)-1-piperazinyl]butyl}-1*H*-imidazole,
74 - 4,5-dichloro-2-methyl-1-{4-[4-(2-fluorophenyl)-1-piperazinyl]butyl}-1*H*-imidazole,
75 - 4-chloro-1-{4-[4-(2-cyanophenyl)-1-piperazinyl]-butyl}-1*H*-pyrazole,
76 - 4,5-dichloro-2-methyl-1-{4-[4-(3-trifluoromethylphenyl)-1-piperazinyl]butyl}-1*H*-imidazole,
77 - 4-chloro-1-{4-[4-(3-trifluoromethylphenyl)-1-piperazinyl]butyl}-1*H*-pyrazole,
78 - 4-chloro-1-{4-[4-(2-fluorophenyl)-1-piperazinyl]-butyl}-1*H*-pyrazole,
79 - 4-chloro-1-{4-[4-(1,2-benzisothiazol-3-yl)-1-piperazinyl]butyl}-1*H*-pyrazole,
80 - 4,5-dichloro-2-methyl-1-{4-[4-(1,2-benzisothiazol-3-yl)-1-piperazinyl]butyl}-1*H*-imidazole,
81 - 1-{4-[4-(1,2-benzisothiazol-3-yl)-1-piperazinyl]-butyl)-1*H*-1,2,4-triazole,
82 - 1-{4-[4-(1,2-benzisothiazol-3-yl)-1-piperazinyl]-butyl}-1*H*-benzimidazole,
83 - 4-bromo-1-{4-[4-(5-bromopyrimidin-2-yl)-1-piperazinyl]butyl}-1*H*-pyrazole,
84 - 4-bromo-1-{4-[4-(5-chloropyrimidin-2-yl)-1-piperazinyl]butyl}-1*H*-pyrazole,

## Patentansprüche

1. Verwendung von Derivaten der allgemeinen Formel I 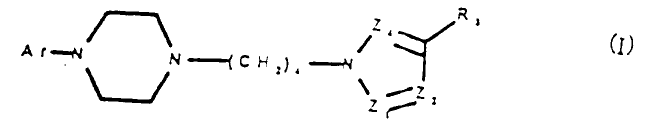 wobei
Ar einen aromatischen stickstoffhaltigen oder nicht stickstoffhaltigen Rest darstellt, der ausgewählt wird aus verschieden substituierten Arylen, unterschiedlich substituiertem 2-Pyrimidin und 3-(1,2-Benzisothiazol),
Z₁ ein Stickstoffatom oder ein substituiertes oder nicht substituiertes Kohlenstoffatom darstellt, das man durch C-R₁ darstellen kann,
Z₂ ein Stickstoffatom oder ein substituiertes oder nicht substituiertes Kohlenstoffatom darstellt, das man durch C-R₂ darstellen kann,
Z₄ ein Stickstoffatom oder ein substituiertes oder nicht substituiertes Kohlenstoffatom darstellt, das man durch C-R₄ darstellen kann,
und R₁, R₂, R₃ und R₄, identisch oder verschieden, die auch einen Teil eines weiteren aromatischen oder nicht aromatischen Rings bilden können, ein Wasserstoffatom, ein Halogen, ein niedriges Alkylradikal, ein Stickstoffradikal, ein Hydroxyradikal, ein Alkoxyradikal, ein Cyanoradikal, ein Carboxylradikal, ein Carboxamidoradikal, ein Alkylcarboxylatradikal, ein Arylradikal oder substituiertes Arylradikal, ein Sulfonylradikal, ein an der Aminogruppe substituiertes oder unsubstituiertes Sulfonaminoradikal, ein Aminoradikal oder substituiertes Aminoradikal darstellen,
und ihre therapeutisch verträglichen Salze zur Herstellung von Medikamenten für die Behandlung von gastrointestinalen Erkrankungen, die hauptsächlich als Inhibitoren der Magensäuresekretion oder als Antiulcusmittel bezeichnet werden.

2. Verwendung nach Anspruch 1, dadurch charakterisiert, daß das Derivat der allgemeinen Formel I ausgewählt wird aus:
1 - 1-{4-[4-(2-Pyrimidinyl)-1-piperazinyl]-butyl}-pyrrol,
2 - 1-{4-[4-(2-Pyrimidinyl)-1-piperazinyl]-butyl}-carbazol,
3 - 1-{4-[4-(2-Pyrimidinyl)-1-piperazinyl]-butyl}-indol,
4 - 2,3-Diphenyl-1-{4-[4-(2-pyrimidinyl)-1-piperazinyl]-butyl}-indol,
5 - 4-Carboxamido-1-{4-[4-(2-pyrimidinyl)-1-piperazinyl]-butyl}-1H-pyrazol,
6 - 4-Carboxy-1-{4-[4-(2-pyrimidinyl)-1-piperazinyl]-butyl}-1H-pyrazol,
7 - 3-Methyl-5-trifluoromethyl-1-{4-[4-(2-pyrimidinyl)-1-piperazinyl]-butyl}-1H-pyrazol,
8 - 4,5-Diphenyl-1-{4-[4-(2-pyrimidinyl)-1-piperazinyl]-butyl}-1H-imidazol,
9 - 2,4,5-Triphenyl-1-{4-[4-(2-pyrimidinyl)-1-piperazinyl]-butyl}-1H-imidazol,
10 - 4,5-Diphenyl-2-methyl-1-{4-[4-(2-pyrimidinyl)-1-piperazinyl]-butyl}-1H-imidazol,
11 - 4,5-Dichloro-2-methyl-1-{4-[4-(2-pyrimidinyl)-1-piperazinyl]-butyl}-1H-imidazol,
12 - 2-Ethyl-1-{4-[4-(2-pyrimidinyl)-1-piperazinyl]-butyl}-1H-imidazol,
13 - 2-Phenyl-1-{4-[4-(2-pyrimidinyl)-1-piperazinyl]-butyl}-1H-imidazol,
14 - 4-Methylcarboxylato-1-{4-[4-(2-pyrimidinyl)-1-piperazinyl]-butyl}-1H-imidazol,
15 - 4-Phenyl-1-{4-[4-(2-pyrimidinyl)-1-piperazinyl]-butyl}-1H-imidazol,
16 - 1-{4-[4-(2-Pyrimidinyl)-1-piperazinyl]-butyl}-1H-benzimidazol,
17 - 1-{4-[4-(2-Pyrimidinyl)-1-piperazinyl]-butyl}-3H-imidazo[5,4-b]pyridin,
18 - 1-{4-[4-(2-Pyrimidinyl)-1-piperazinyl]-butyl}-1H-imidazo[4,5-b]pyridin,
19 - 1-{4-[4-(2-Pyrimidinyl)-1-piperazinyl]-butyl}-1H-benzotriazol,
20 - 2-Chloro-1-{4-[4-(2-pyrimidinyl)-1-piperazinyl]-butyl}-1H-benzimidazol,
21 - 1-{4-[4-(2-Pyrimidinyl)-1-piperazinyl]-butyl}-1H-1,2,4-triazol,
22 - 2-{4-[4-(2-Pyrimidinyl)-1-piperazinyl]-butyl}-2H-benzotriazol,
23 - 2-Methyl-1-{4-[4-(2-pyrimidinyl)-1-piperazinyl]-butyl}-1H-benzimidazol,
24 - 5,6-Dimethyl-1-{4-[4-(2-pyrimidinyl)-1-piperazinyl]-butyl}-1H-benzimidazol,
25 - 1-{4-[4-(2-pyrimidinyl)-1-piperazinyl]-butyl}-1H-pyrazol,
26 - 3,5-Dimethyl-1-{4-[4-(2-pyrimidinyl)-1-piperazinyl]-butyl}-1H-pyrazol,
27 - 3,5-Dimethyl-4-nitro-1-{4-[4-(2-pyrimidinyl)-1-piperazinyl]-butyl}-1H-pyrazol,
28 - 4-Methyl-1-{4-[4-(2-pyrimidinyl)-1-piperazinyl]-butyl}-1H-pyrazol,
29 - 1-{4-[4-(2-pyrimidinyl)-1-piperazinyl]-butyl}-1H-indazol,
30 - 4-Bromo-3,5-dimethyl-1-{4-[4-(2-pyrimidinyl)-1-piperazinyl]-butyl}-1H-pyrazol,
31 - 4-Nitro-1-{4-[4-(2-pyrimidinyl)-1-piperazinyl]-butyl}-1H-pyrazol,
32 - 4-Chloro-1-{4-[4-(2-pyrimidinyl)-1-piperazinyl]-butyl}-1-H-pyrazol dihydrochlorid,
33 - 4-Ethylcarboxylato-1-{4-[4-(2-pyrimidinyl)-1-piperazinyl]-butyl}-1-H-pyrazol,
34 - 3-Methyl-5-phenyl-1-{4-[4-(2-pyrimidinyl)-1-piperazinyl]-butyl}-1H-pyrazol,
35 - 4-Bromo-1-{4-[4-(2-pyrimidinyl)-1-piperazinyl]-butyl}-1H-pyrazol,
36 - 4-Cyano-1-{4-[4-(2-pyrimidinyl)-1-piperazinyl]-butyl}-1H-pyrazol,
37 - 4-Fluoro-1-{4-[4-(2-pyrimidinyl)-1-piperazinyl]-butyl}-1H-pyrazol,
38 - 4-Amino-1-{4-[4-(2-pyrimidinyl)-1-piperazinyl]-butyl}-1H-pyrazol,
39 - 4-Methylsulfonamido-1-{4-[4-(2-pyrimidinyl)-1-piperazinyl]-butyl}-1H-pyrazol,
40 - 4-Benzamido-1-{4-[4-(2-pyrimidinyl)-1-piperazinyl]-butyl}-1H-pyrazol,
41 - 4-Acetamido-1-{4-[4-(2-pyrimidinyl)-1-piperazinyl]-butyl}-1H-pyrazol,
42 - 4-(2-Butyl)amino-1-{4-[4-(2-pyrimidinyl)-1-piperazinyl]-butyl}-1H-pyrazol,
43 - 3-Chloro-4-fluoro-1-{4-[4-(2-pyrimidinyl)-1-piperazinyl]-butyl}-1H-pyrazol,
44 - 4-(4-Methoxyphenyl)-1-{4-[4-(2-pyrimidinyl)-1-piperazinyl]-butyl}-1H-pyrazol,
45 - 4-(4-Chlorophenyl)-1-{4-[4-(2-pyrimidinyl)-1-piperazinyl]-butyl}-1H-pyrazol,
46 - 4-(1-Pyrrolyl)-1-{4-[4-(2-pyrimidinyl)-1-piperazinyl]-butyl}-1H-pyrazol,
47 - 4-Phenyl-1-{4-[4-(2-pyrimidinyl)-1-piperazinyl]-butyl}-1H-pyrazol,
48 - 3,5-Diphenyl-1-{4-[4-(2-pyrimidinyl)-1-piperazinyl]-butyl}-1H-pyrazol,
49 - 4-Phenylsulfamoyl-1-{4-[4-(2-pyrimidinyl)-1-piperazinyl]-butyl}-1H-pyrazol,
50 - 4-(4-Methylphenyl)sulfamoyl-1-{4-[4-(2-pyrimidinyl)-1-piperazinyl]-butyl}-1H-pyrazol,
51 - 4-Butylsulfamoyl-1-{4-[4-(2-pyrimidinyl)-1-piperazinyl]-butyl}-1H-pyrazol,
52 - 4-Propylsulfamoyl-1-{4-[4-(2-pyrimidinyl)-1-piperazinyl]-butyl}-1H-pyrazol,
53 - 4-Ethylsulfamoyl-1-{4-[4-(2-pyrimidinyl)-1-piperazinyl]-butyl}-1H-pyrazol,
54 - 3,5-Dimethyl-4-(N,N-dimethylsulfonamido)-1-{4-[4-(2-pyrimidinyl)-1-piperazinyl]-butyl}-1H-pyrazol,
55 - 4-N-Methylsulfamoyl-1-{4-[4-(2-pyrimidinyl)-1-piperazinyl]-butyl}-1H-pyrazol,
56 - 4-Sulfonyl-1-{4-[4-(2-pyrimidinyl)-1-piperazinyl]-butyl}-1H-pyrazol,
57 - 1-{4-[4-(2-pyrimidinyl)-1-piperazinyl]-butyl}-1H-imidazol,
58 - 2-Methyl-1-{4-[4-(2-pyrimidinyl)-1-piperazinyl]-butyl}-1H-imidazol,
59 - 4,5-Dichloro-1-{4-[4-(2-pyrimidinyl)-1-piperazinyl]-butyl}-1H-imidazol,
60 - 4-Chloro-1-{4-[4-(4-methoxyphenyl)-1-piperazinyl]-butyl}-1H-pyrazol,
61 - 4,5-Dichloro-2-methyl-1-{4-[4-(4-methoxyphenyl)-1-piperazinyl]-butyl}-1H-imidazol,
62 - 4-Chloro-1-{4-[4-(2-methoxyphenyl)-1-piperazinyl]-butyl}-1H-pyrazol,
63 - 4,5-Dichloro-2-methyl-1-{4-[4-(2-methoxyphenyl)-1-piperazinyl]-butyl}-1H-imidazol,
64 - 4-Chloro-1-{4-[4-(3-methoxyphenyl)-1-piperazinyl]-butyl}-1H-pyrazol,
65 - 1-{4-[4-(4-Methoxyphenyl)-1-piperazinyl]-butyl}-pyrrol,
66 - 1-{4-[4-(2-Methoxyphenyl)-1-piperazinyl]-butyl}-pyrrol,
67 - 1-{4-[4-(Phenyl)-1-piperazinyl]-butyl}-pyrrol,
68 - 4-Chloro-1-{4-[4-(phenyl)-1-piperazinyl]-butyl}-1H-pyrazol,
69 - 4,5-Dichloro-2-methyl-1-{4-[4-(phenyl)-1-piperazinyl]-butyl}-1H-imidazol,
70 - 4-Chloro-1-{4-[4-(2-chlorophenyl)-1-piperazinyl]-butyl}-1H-pyrazol,
71 - 4,5-Dichloro-2-methyl-1-{4-[4-[(2-chlorophenyl)-1-piperazinyl[-butyl}-1H-imidazol,
72 - 4-Chloro-1-{4-[4-(3-chlorophenyl)-1-piperazinyl)-butyl}-1H-pyrazol,
73 - 4,5-Dichloro-2-methyl-1-{4-[4-(2-cyanophenyl-1-piperazinyl]-butyl}-1H-imidazol,
74 - 4,5-Dichloro-2-methyl-1-{4-[4-(2-fluorophenyl)-1-piperazinyl]-butyl}-1H-imidazol,
75 - 4-Chloro-1-{4-[4-(2-cyanophenyl)-1-piperazinyl]-butyl}-1H-pyrazol,
76 - 4,5-Dichloro-2-methyl-1-{4-[4-(3-trifluoromethylphenyl)-1-piperazinyl]-butyl}-1H-imidazol,
77 - 4-Chloro-1-{4-[4-(3-trifluoromethylphenyl)-1-piperazinyl]-butyl}-1H-pyrazol,
78 - 4-Chloro-1-{4-[4-(2-fluorophenyl)-1-piperazintyl]-butyl}-pyrazol,
79 - 4-Chloro-1-{4-[4-(1,2-benzisothiazol-3-yl)-1-piperazinyl]-butyl}-1H-pyrazol,
80 - 4,5-Dichloro-2-methyl-1-{4-[4-(1,2-benzisothiazol-3-yl)-1-piperazinyl]-butyl}-1H-imidazol,
81 - 1-{4-[4-(1,2-Benzisothiazol-3-yl)-1-piperazinyl]-butyl}-1H-1,2,4-triazol,
82 - 1-{4-[4-(1,2-Benzisothiazol-3-yl)-1-piperazinyl]-butyl}-1H-benzimidazol,
83 - 4-Bromo-1-{4-[4-(5-bromopyrimidin-2-yl)-1-piperazinyl]-butyl}-1H-pyrazol,
84 - 4-Bromo-1-{4-[4-(5-chloropyrimidin-2-yl)-1-piperazinyl]-butyl}-1H-pyrazol
